(19) Europäisches Patentamt / European Patent Office / Office européen des brevets

(11) **EP 4 516 272 A1**

(12) **EUROPEAN PATENT APPLICATION**
published in accordance with Art. 153(4) EPC

(43) Date of publication:
**05.03.2025 Bulletin 2025/10**

(21) Application number: **23920031.4**

(22) Date of filing: **28.12.2023**

(51) International Patent Classification (IPC):
**A61F 5/453** (2006.01)

(52) Cooperative Patent Classification (CPC):
**A61F 5/453**

(86) International application number:
**PCT/JP2023/047253**

(87) International publication number:
**WO 2024/161896 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.01.2023 JP 2023013553
28.04.2023 JP 2023075234**

(71) Applicant: **Intron Space Inc.
Tokyo, 116-0003 (JP)**

(72) Inventors:
• **IMAI Shigeo**
  **Tokyo 116-0003 (JP)**
• **KONDO Takashi**
  **Tokyo 116-0003 (JP)**
• **MYOCHIN Mitsumasa**
  **Tokyo 116-0003 (JP)**
• **TAMAOKI Toshiaki**
  **Tokyo 116-0003 (JP)**
• **OKABE Yusuke**
  **Tokyo 116-0003 (JP)**

(74) Representative: **Canzler & Bergmeier
Patentanwälte
Partnerschaft mbB
Despag-Straße 6
85055 Ingolstadt (DE)**

(54) **BIOFLUID ACCOMMODATION CONTAINER, AND CONTAINER BODY, SEALING BODY, AND FALLING PREVENTION BODY USED THEREIN**

(57)    Provided is a biofluid accommodation container that can be worn and carried on a body, has a simple configuration, is comfortable to wear, does not leak urine during urine collection, and has an accommodation quantity that can correspond to the urination quantity. In a container body 100, a hollow accommodation part 111 that accommodates a biofluid BF, a hollow introduction part 112 that is provided with an introduction section, which is in communication with one end of the accommodation part and introduces the biofluid, a hollow discharge part 113 that is provided with a discharge section, which is in communication with the other end of the accommodation part and discharges the biofluid, a hollow guidance part 114 that is in communication with the opposite side of the introduction part from an introduction opening section, is oriented toward the accommodation part, and guides the biofluid into the accommodation part, and a check valve 115 that is formed in the guidance part and prevents reverse flow of the biofluid are all formed as a single body from a material that has a low hardness and a high distensibility, and the accommodation part 111 comprises a high distensibility section that has a higher distensibility than the distensibility of any section of the introduction part and the discharge part.

EP 4 516 272 A1

[Fig. 4]

**Description**

Technical Field

The present invention relates to a biological fluid receptacle container and a container body, a closure member, and a detachment prevention member used therefor, and particularly to a container for receiving urine excreted from a human body and a detachment prevention member used for the penis.

Background Art

Conventionally, as a biological fluid receptacle container, there are known a male urination device which prevents urine from leaking out of a gap between an external attachment portion and the penis by means of a ring-shaped adhesive pad attached closely to the glans corona (see Patent Literature 1), a female urination tool in which a urine receptacle bag is attached to an opening portion of clothing by means of a band provided with a ring (see Patent Literature 2), a urination device in which a penis insertion bag integrated with a water-repellent film and a urine receptacle container are connected to each other through a pipe having a built-in check valve (see Patent Literature 3), a small urine collecting container which has a built-in check valve and can be attached under clothing (see Patent Literature 4), a urination device in which through a tube attached to the penis, excreted urine flows down into a collecting container attached to the crotch and is collected therein, and the urine is discharged from a valve of the collecting container (see Patent Literature 5), a condom which is configured to be attached in a collapsed state to the tip end of the glans and has a semen reception bag expandable in accordance with ejaculation (see Patent Literature 6), a tubular semen collector made of a flexible material which is thick near an inlet and thin at a middle portion (see Patent Literature 7), and a urine receptacle container in which an outlet of an attachment portion for covering the penis and a receptacle container are connected to each other through a tube provided with a check valve (see Patent Literature 8).

Prior Art Documents

Patent Literature

Patent Literature 1: JPS58-1445A
Patent Literature 2: JP2016-52393A
Patent Literature 3: JP2000-157566A
Patent Literature 4: JP2004-57747A
Patent Literature 5: US2005/251100A1
Patent Literature 6: US5458114A
Patent Literature 7: JP3146490U
Patent Literature 8: JP2021-74491A

Summary of Invention

Technical Problem

However, these conventional biological fluid receptacle containers have such problems that the biological fluid receptacle container is not portable while being attached to the human body, and a device is made of a hard material and thus uncomfortable to wear (cited document 1), the structure is complicated (cited documents 1, 2, 3), urine leakage may occur during urine collection (cited documents 3, 4), an amount of urine collected may exceed a capacity (cited documents 2, 3, 4, 5), and the biological fluid receptacle container is not suitable for receiving urine (cited documents 6, 7).

To comprehensively solve these problems, the applicant filed a patent application for an invention relating to a urine receptacle container prior to the present application (see Patent Literature 8), which, however, did not disclose a specific structure of an attachment portion for an excretory organ of a human body.

As a result of the subsequent continued study, for a receptacle container for a biological fluid, specifically urine, the inventors developed a biological fluid receptacle container including a container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at this guide portion and configured to prevent backflow of the biological fluid, and a closure member made of a flexible material.

While studying the invention relating to this novel biological fluid receptacle container in detail, the inventors have discovered the following new problems of the biological fluid receptacle container which should be further solved.

<Problem of the container body>

When the container body of the biological fluid receptacle container is integrally formed from a material having a low hardness and a high elongation, (1) an attachment portion for the penis is easily deformed due to a high flexibility and a high elongation, so that the attachment portion is easily detached from the penis, further, (2) the guide portion is easily deformed, so that an opening position of a check valve may be displaced from the external urethral orifice, which may block the external urethral orifice and urine may not be properly guided to the receptacle portion, moreover, (3) the check valve portion and the guide portion are easily deformed, so that when a pressure is applied to the receptacle portion from the exterior, the check valve portion and the guide portion may turn over, which may cause urine to flow back, furthermore, (4) with a conventional structure of a check valve, such as a duckbill valve and an umbrella valve, when a urine pressure is low, the check valve may not open and urine may not be properly guided to the receptacle portion, and in addition, (5) when the receptacle portion is configured to be held at a higher position than the introduction portion, urine may not be properly guided to the receptacle portion due to the influence of gravity.

<Problem of the closure member>

(6) When the closure member is made of a flexible material in order to improve the wearing comfort, then deformation of the closure member may cause insufficient closure of the outlet port of the container body to result in urine leakage.

<Problem of the detachment prevention member>

(7) When a tubular detachment prevention member is attached to the penis in order to prevent the container body from detaching, then urination may be hindered due to a pressure, further, when the detachment prevention member is attached to the penis having loose foreskin, (8) the foreskin returns toward the tip end of the glans, which causes the glans to relatively move from the large diameter region to the introduction port, thereby creating a space around the large diameter region, so that the penis may slip out of the container body, and moreover, (9) the foreskin moves toward the tip end of the penis together with the detachment prevention member to cover the entirety of the glans, which may hinder urination and urine collection.

Thus, the present invention aims to solve the problems of the prior art, and aims further to also solve the new problems that the inventors have found in the course of development.

In other words, it is a first object of the present invention to provide a biological fluid receptacle container which is portable while being attached to the human body, has a simple structure, is comfortable to wear, does not leak urine during urine collection, and is adaptable to large and small amounts of urination.

Further, it is a second object of the present invention to provide a container body for a biological fluid receptacle container in which the container body does not easily detach from the penis even when being integrally formed from a material having a low hardness and a high elongation, excreted urine is reliably guided to the receptacle portion, backflow of urine due to an external pressure does not occur, urine can be properly guided to the receptacle portion even when a urine pressure is low, and urine is guided to the receptacle portion even when the receptacle portion is held at a position higher than the introduction portion.

Moreover, it is a third object of the present invention to provide a closure member for a biological fluid receptacle container which reliably prevents urine leakage even when being made of a flexible material for better comfort to wear, and allows urine to be discharged through an easy operation.

In addition, it is a fourth object of the present invention to provide a detachment prevention member which prevents such inconvenience that the penis slips out of the container body during use of a biological fluid receptacle container, and does not hinder urination and urine collection even when being attached to the penis. Solution to Problem

The invention according to claim 1 is to solve the above problems by a container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, in which the receptacle portion has such a flat shape as to have a bulge part in a lateral direction, and the check valve portion forms a valve opening having a slit shape such that substantially a lateral direction is a longitudinal direction.

The invention according to claim 2 is to solve the above problems by a container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, in which a support part of the guide portion includes a large diameter region and a small diameter region formed at a position closer to the introduction portion than the large diameter region, and a receiving space bottom part of the receptacle portion is arranged at a position corresponding to the small diameter region.

The invention according to claim 3 is to further solve the above problems by a container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, in which a minimum thickness dimension of the guide portion is configured to be smaller than a minimum thickness dimension of the introduction portion.

The invention according to claim 4 is to further solve the above problems by, in addition to the configuration of the invention according to any one of claim 1 to claim 3, a feature in which the receptacle portion includes a high elongation region having an elongation higher than an elongation of any region of the introduction portion and the outlet portion.

The invention according to claim 5 is to solve the above problems by, in addition to the configuration of the invention according to any one of claim 1 to claim 3, a feature in which the receptacle portion, the guide portion, and the outlet portion are made of the same material, and a minimum thickness dimension of the receptacle portion is configured to be smaller than any of a minimum thickness dimension of the guide portion and a minimum thickness dimension of the introduction portion.

The invention according to claim 6 is to solve the above problems by, in addition to the configuration of the invention according to any one of claim 1 to claim 3, a feature in which the check valve portion includes a pair of valve membrane parts arranged facing each other and toward the receptacle portion at respective positions close to each other in a manner separate from each other.

The invention according to claim 7 is to solve the above problems by, in addition to the configuration of the invention according to any one of claim 1 to claim 3, a feature in which the guide portion is configured to have a length dimension larger than half an inner diameter dimension of an introduction channel formed in the hollow introduction portion.

The invention according to claim 8 is to solve the above problems by a detachment prevention member having a tubular shape, the detachment prevention member being configured to be used together with a biological fluid receptacle container including: a container body comprising at least a hollow receptacle portion configured to receive a biological fluid and a hollow introduction portion configured to communicate with one side of the receptacle portion and introduce the biological fluid; and a closure member, the detachment prevention member being configured by integrally forming from a flexible material: a front covering portion including a semi-cylindrical front outer surface part and a semi-cylindrical front inner surface part, the front covering portion having a semi-cylindrical shape having a fixed thickness; and a back covering portion including a flat back outer surface part and a back inner surface part having a substantially V-shaped cross-sectional shape, the back covering portion having such a shape that a thickness thereof decreases toward a center.

The invention according to claim 9 is to solve the above problems by, in addition to the configuration of the invention according to claim 8, a feature in which the front covering portion has a foreskin accommodation window part configured to communicate from the front inner surface part to the front outer surface part.

Effects of Invention

According to the container body of the invention according to claim 1 to claim 3, a feature of integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid not only realizes a biological fluid receptacle container which is portable while being attached to the human body, has a simple structure, is comfortable to wear, does not leak urine during urine collection, and is adaptable in capacity to large and small amounts of urination, but also exhibits the following effects inherent to the invention of the present application on the basis of the following configurations inherent to the invention of the present application.

According to the container body of the invention according to claim 1, the receptacle portion has such a flat shape as to have a bulge part in a lateral direction, and the check valve portion forms a valve opening having a slit shape such that substantially a lateral direction is a longitudinal direction, which realizes an arrangement during attachment to the body such that a major axis direction of the valve opening of the check valve is substantially perpendicular to a major axis direction of the external urethral orifice so that the external urethral orifice overlaps with the check valve opening, which can prevent blockage of the external urethral orifice caused by an offset of the valve opening of the check valve from the external urethral orifice during attachment.

According to the container body of the invention according to claim 2, a support part of the guide portion includes a large diameter region and a small diameter region formed at a position closer to the introduction portion than the large diameter region, and a receiving space bottom part of the receptacle portion is arranged at a position corresponding to the small diameter region, which generates a force of pushing the small diameter region of the guide portion from the outside to the inside at the receiving space bottom part in accordance with an increase of an internal pressure of the biological fluid received in the receptacle portion so as to increase a force of holding the penis, so that the penis does not slip out of the introduction portion and accordingly, detachment of the container body can be prevented.

According to the container body of the invention according to claim 3, a minimum thickness dimension of the guide portion is configured to be smaller than a minimum thickness dimension of the introduction portion, whereby when the biological fluid receptacle container receives a biological fluid more than or equal to an initial capacity, the guide portion expands earlier than the introduction portion, which can prevent detachment of the container body caused by earlier expansion of the guide portion than the introduction portion and slipping of the penis out of the guide portion.

According to the container body of the invention according to claim 4, in addition to the effects produced by the invention according to any one of claim 1 to claim 3, the receptacle portion includes a high elongation region having an elongation higher than an elongation of any region of the introduction portion and the outlet portion, whereby when the container body receives a biological fluid more than or equal to an initial capacity, a region having a higher elongation from the receptacle portion expands earlier than the other regions, which can prevent inconvenience, such as detachment caused by earlier expansion of the guide portion and resulting slipping of the glans out of the guide portion, and leakage of a biological fluid caused by earlier expansion of the outlet portion.

According to according to claim 5, in addition to the effects produced by the invention according to any one of claim 1 to claim 3, the receptacle portion, the guide portion, and the outlet portion are made of the same material, and a minimum thickness dimension of the receptacle portion is configured to be smaller than a minimum thickness dimension of the guide portion, whereby the receptacle portion includes a high elongation region having a higher elongation than the guide portion and when the biological fluid receptacle container receives a biological fluid more than or equal to an initial capacity, the receptacle portion expands earlier than the guide portion, which can prevent reception incapability caused by expansion of the guide portion while the check valve portion is closed due to an internal pressure of the receptacle portion.

Further, the receptacle portion, the guide portion, and the outlet portion are made of the same material, and a minimum thickness dimension of the receptacle portion is configured to be smaller than a minimum thickness dimension of the introduction portion, whereby the receptacle portion includes a high elongation region having a higher elongation than the introduction portion and when the biological fluid receptacle container receives a biological fluid more than or equal to an initial capacity, the receptacle portion expands earlier than the introduction portion, which can prevent detachment of the container body caused by earlier expansion of the introduction portion than the receptacle portion and slipping of the penis out of the introduction portion.

According to the container body according to claim 6, in addition to the effects produced by the invention according to any one of claim 1 to claim 3, the check valve portion includes a pair of valve membrane parts arranged facing each other and toward the receptacle portion at respective positions close to each other in a manner separate from each other, whereby when a direct flow in which a biological fluid flows from the introduction portion toward the receptacle portion occurs, the valve opening of the check valve further expands due to an elongation, so that the biological fluid can be efficiently guided into the receptacle portion. Further, when a reverse flow in which a biological fluid flows from the receptacle portion toward the guide portion occurs due a pressure from the exterior or the like, the valve opening of the check valve portion immediately closes due to a flexibility, so that an occurrence of a reverse flow can be reliably prevented.

According to the container body according to claim 7, in addition to the effects produced by the invention according to any one of claim 1 to claim 3, the guide portion is configured to have a length dimension larger than half an inner diameter dimension of an introduction channel formed in the hollow introduction portion, whereby while a biological fluid is received in the receptacle portion, the guide portion is pushed toward the introduction portion by an internal pressure of the biological fluid and thus deformed so as to block the introduction channel of the introduction portion configured to communicate with the guide portion, so that even when the introduction portion unexpectedly detaches from the penis during use, backflow and leakage of the received biological fluid can be prevented.

According to the detachment prevention member of the invention according to claim 8, the detachment prevention member has a tubular shape, the detachment prevention member being configured to be used together with a biological fluid receptacle container including: a container body comprising at least a hollow receptacle portion configured to receive a biological fluid and a hollow introduction portion configured to communicate with one side of the receptacle portion and introduce the biological fluid; and a closure member, whereby when the penis is inserted and attached into the detachment prevention member, an outer diameter of the penis is substantially increased, so that a force of holding the penis in the introduction portion increases, the penis does not slip out of the introduction portion and accordingly, detachment of the container body can be prevented.

Further, the detachment prevention member is configured by integrally forming from a flexible material: a front covering portion including a semi-cylindrical front outer surface part and a semi-cylindrical front inner surface part, the front covering portion having a semi-cylindrical shape having a fixed thickness; and a back covering portion including a flat back outer surface part and a back inner surface part having a substantially V-shaped cross-sectional shape, the back covering portion having such a shape that a thickness thereof decreases toward a center, thereby relieving a pressure on the urethra during attachment to the penis, and further, when the container body is attached while the detachment prevention member is attached to the penis, a pushing force of the introduction portion from the outside to the inside causes a central bent part of the back covering portion of the detachment prevention member to bend outward such that a pressure on the urethra continues to be relieved, so that it is possible to prevent the container body from detaching without hindering prompt urination or, further, without slipping of the penis out of the introduction portion.

According to the detachment prevention member of the invention according to claim 9, in addition to the effects produced by claim 8, the front covering portion has a foreskin accommodation window part configured to communicate from the front inner surface part to the front outer surface part, whereby when the detachment prevention member is attached to the penis, the loose foreskin is fixed in such a manner as to remain within the foreskin accommodation window part, so that when the detachment prevention member is attached to the penis having the loose foreskin, it is possible to prevent such inconvenience that the foreskin returns toward the tip end of the glans, which causes the glans to relatively move from the large diameter region of the container body to the introduction port part, thereby creating a space near the large diameter region, so that the penis slips out of the container body, and further, it is possible to prevent such inconvenience that the foreskin moves toward the tip end of the penis together with the detachment prevention member and covers the entire glans to hinder urination and urine collection.

Further, the front covering portion has a foreskin accommodation window part configured to communicate from the front inner surface part to the front outer surface part, so that it is unnecessary to forcibly lower the foreskin toward the base of the penis to attach the detachment prevention member, which can reduce user discomfort.

Brief Description of Drawings

Fig. 1 is a schematic diagram illustrating a state in which a user wears on the penis a biological fluid receptacle container according to a first embodiment of the present invention.
Fig. 2A is a schematic front view illustrating a container body of the biological fluid receptacle container according to the first embodiment of the present invention.
Fig. 2B is a schematic side view illustrating the container body of the biological fluid receptacle container according to the first embodiment of the present invention.
Fig. 3 is a schematic cross-sectional view of fig. 1 along III-III.
Fig. 4 is a schematic perspective view illustrating the biological fluid receptacle container according to the first embodiment of the present invention.
Fig. 5A is a schematic front view illustrating a state in which urine is received in a receptacle portion using the biological fluid receptacle container according to the first embodiment of the present invention.
Fig. 5B is a schematic side view illustrating a state in which urine is received in the receptacle portion using the biological fluid receptacle container according to the first embodiment of the present invention.
Fig. 6A is a schematic diagram illustrating a state of discharging a biological fluid received in the biological fluid receptacle container according to the first embodiment of the present invention.

Fig. 6B is a schematic diagram illustrating a state of adjusting a discharge direction of the biological fluid received in the biological fluid receptacle container according to the first embodiment of the present invention.

Fig. 7A is a schematic diagram illustrating a first example of a state in which the biological fluid receptacle container according to the first embodiment of the present invention has detached from the penis during use.

Fig. 7B is a schematic diagram illustrating a second example of a state in which the biological fluid receptacle container according to the first embodiment of the present invention has detached from the penis during use.

Fig. 8 is a schematic perspective view illustrating a container body according to a second embodiment of the present invention.

Fig. 9 is a schematic diagram illustrating a state in which a receptacle portion according to a third embodiment of the present invention includes a plurality of sets of guide portions and check valve portions.

Fig. 10A is a schematic diagram illustrating a state in which a receptacle portion according to a fourth embodiment of the present invention includes a plurality of sets of guide portions and check valve portions.

Fig. 10B is a schematic diagram illustrating a state in which a biological fluid is moved upward in the receptacle portion illustrated in fig. 10A.

Fig. 11 is a diagram illustrating a closure member according to a first reference example of the present invention.

Fig. 12A is a schematic cross-sectional view illustrating a first example of a cross section of fig. 11 along XII-XII.

Fig. 12B is a schematic cross-sectional view illustrating a second example of a cross section of fig. 11 along XII-XII.

Fig. 12C is a schematic cross-sectional view illustrating a third example of a cross section of fig. 11 along XII-XII.

Fig. 13A is a diagram illustrating a closure member according to a second reference example of the present invention.

Fig. 13B is a diagram illustrating a state in which a closure valve portion of the closure member illustrated in fig. 13A is open.

Fig. 14 is a cross-sectional view of fig. 13A along XIV-XIV.

Fig. 15 is a diagram illustrating a closure member according to a third reference example of the present invention.

Fig. 16 is a plan view of the closure member illustrated in fig. 15.

Fig. 17 is a cross-sectional view of fig. 16 along XVII-XVII.

Fig. 18 is a cross-sectional view of fig. 16 along XVIII-XVIII.

Fig. 19 is a schematic diagram illustrating a closure member according to a fourth reference example of the present invention.

Fig. 20 is a schematic cross-sectional view of fig. 19 along XX-XX.

Fig. 21 is a schematic diagram illustrating a state in which a detachment prevention member according to a fifth embodiment of the present invention is attached to the penis and then a biological fluid receptacle container is attached.

Fig. 22A is a schematic diagram of the detachment prevention member illustrated in fig. 21 as seen from an insertion direction.

Fig. 22B is a schematic side view of the detachment prevention member illustrated in fig. 21.

Fig. 23 is a schematic cross-sectional view of fig. 21 along XXIII-XXIII.

Fig. 24A is a schematic perspective view of a detachment prevention member according to a sixth embodiment of the present invention.

Fig. 24B is a schematic plan view of the detachment prevention member illustrated in fig. 24A.

Fig. 24C is a schematic side view of the detachment prevention member illustrated in fig. 24A.

Fig. 25 is a schematic diagram illustrating a state in which the detachment prevention member illustrated in fig. 24A is attached to the penis.

Description of Embodiments

First, definitions of terms for explaining embodiments to carry out the present invention will be described.

<Definition of "biological fluid">

In this specification, the term "biological fluid" refers to a fluid which includes water, such as a solution, a suspension, or an emulsion that exists in a living body or is discharged from or emitted by a living body, such as blood, lymph, a tissue fluid or a body cavity fluid of an animal, or a xylem fluid or a phloem fluid of a plant, and is used as a concept which includes, in particular, human urine, menstrual blood or feces.

<Definition of "hardness">

In this specification, the "hardness" means a hardness value measured in accordance with "JIS K 6253". Since the material used for the container body of the biological fluid receptacle container according to the present invention has an extremely low hardness, measurement is carried out using a type E durometer (spring type hardness meter). It is meant that the higher this measured value is, the higher the hardness is (in other words, it is hard and difficult to deform).

Therefore, when the container body of the biological fluid receptacle container is configured using a certain material, a physical property is indicated such that the lower the hardness of the material is, the more flexible and easily deformable the material is.

<Definition of "elongation">

In this specification, the "elongation" refers to a measured value of a single material or a composite material, $1/\sigma \times T$ (millimeter/newton)

where T (millimeter) is the thickness of the material, and $\sigma$ (Newton/square millimeter) is the tensile stress at 70% elongation of the material measured in accordance with "JIS K 6251". The larger this value is, the greater the elongation per unit pressure is, that is, the easier it is to elongate. Further, a "material having a high elongation" refers to a material having a greater elongation when compared at the same thickness.

<Definition of "degree of elongation">

In this specification, the "degree of elongation" refers to the "elongation" measured in accordance with "JIS K 6251" and is a value expressed as a ratio (unit: %) to the test length before the test.

When the container body of the biological fluid receptacle container is made of a single material, a physical property is indicated such that the higher the elongation of the material is, the greater the elongation per unit pressure is and the easier it is to expand due to the internal pressure of the received biological fluid.

When the container body of the biological fluid receptacle container is made of a composite material, a physical property is indicated such that the higher the elongation of the composite material is, the greater the elongation per unit pressure is and the easier it is to expand due to the internal pressure of the received biological fluid.

Consequently, whether it is a single material or a composite material, a physical property is indicated such that the higher the elongation of the material is, the greater the elongation per unit pressure is and the easier it is to expand due to the internal pressure of the received biological fluid.

<Definition of "minimum thickness dimension">

In this specification, the "minimum thickness dimension" refers to the smallest value among the constituent units when the thickness dimensions of each region for each part of the constituent units, namely the receptacle portion, the introduction portion, the outlet portion, and the guide portion, which constitute the container body of the biological fluid receptacle container are measured. For example, the minimum thickness dimension of the receptacle portion refers to the smallest value among the thickness dimensions of each region of the receptacle portion.

Note that for the detachment prevention member, the "minimum thickness dimension" refers to the smallest value when the thickness dimension of each region of the detachment prevention member is measured.

When the container body according to the present invention is integrally formed from the same material, the region indicating the minimum thickness dimension indicates the maximum elongation.

<Definition of initial capacity>

In this specification, the "initial capacity" refers to the capacity in a natural state where the receptacle portion has not been elongated by the internal pressure or the like. Therefore, when the biological fluid more than or equal to the initial capacity is received in the receptacle portion of the container body of the biological fluid receptacle container made of a material having the elongation property, the material constituting the receptacle portion elongates to cause the receptacle portion to expand and increase in capacity, and the internal pressure of the biological fluid increases.

<Preferred embodiments>

Any specific embodiment of the present invention may be suitable as long as the container body of the biological fluid receptacle container according to the present invention is configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, the container body does not easily detach from the penis, excreted urine is reliably guided to the receptacle portion, backflow of urine due to an external pressure does not occur, urine can be properly guided to the receptacle portion even when a urine pressure is low, and urine can be guided to the receptacle portion even when the receptacle portion is held at a position higher than the introduction portion.

Further, the container body according to the present invention may be made of a material having a low hardness, for example, an elastomer having an ultra-low hardness of 20 degrees or less.

Note that elastomers having a hardness of 0 degrees or more and 20 degrees or less are less hard than the skin at soft regions of the human body and can accordingly reduce the discomfort during attachment to the human body in a portable manner, and therefore can be preferably used.

Further, elastomers having a hardness of 0 degrees or more and 10 degrees or less are less hard than the skin at soft regions of the human body and can accordingly reduce the discomfort during attachment to the human body in a portable manner, and therefore can be preferably used.

Note that a measurement method for obtaining a hardness in accordance with a definition different from the definitions of the hardness as described above includes the hardness obtained by measurement using an Asker rubber hardness tester type F as a durometer. According to this measurement method, a value of 0 or more can be obtained for ultra-low hardness materials, and in this measurement method, it is more preferable to use an elastomer having a hardness of 20 to 80.

Elastomers having an elongation of 100% or more allow the receptacle portion of the container body to expand in accordance with reception of a biological fluid and can increase the capacity, and therefore can be preferably used.

In particular, elastomers having an elongation of 2000% to 3000% do not substantially restrict the capacity of the receptacle portion and have such a sufficient elongation as to allow for long-term use and reception of large amounts of biological fluids, and therefore can be more preferably used.

Examples of materials which satisfy the above conditions of both the hardness and the elongation may include a thermoplastic elastomer (TPE), such as a styrene-based, olefin-based, urethane-based, ester-based, PVC-based or amide-based TPE, and in particular, KURAIN (registered trademark) manufactured by Kuraray Trading Co., Ltd. which is made of a compound containing at least five times paraffin oils in a styrene-based thermoplastic elastomer can be preferably used.

Note that the human skin exhibits an elongation ratio (ratio of elongation during normal movement) of approximately 70 percent at a maximum, so that elastomers having an elongation ratio of more than 70 percent do not cause pain or a tight sensation to the user during attachment to the human body and therefore can be preferably used.

The biological fluid receptacle container according to the present invention can receive various biological fluids, i.e., a fluid which includes water, such as a solution, a suspension, or an emulsion that exists in a living body or is discharged or derived from a living body, such as blood, lymph, a tissue fluid or a body cavity fluid of an animal, or a xylem fluid or a phloem fluid of a plant, and can receive, in particular, human urine, menstrual blood or feces.

Since these biological fluids include those excreted from the excretory organ of animals including humans, those that flow out from wounds, such as cuts, abrasions, contusions, crush wounds, puncture wounds, bites, burns, electric shock wounds, and bedsores in animals or plants, and those that are emitted via a catheter inserted into the living body of an animal or plant out of the body, there can be various configurations of the biological fluid receptacle container depending upon the type, amount, physical property, introduction method or the like of the biological fluids.

For example, to receive urine excreted from the penis which is a male excretory organ, the introduction portion of the container body can have a hollow shape such that the introduction port part for introducing urine is provided and the introduction channel having a circular cross section is formed in the interior of the tubular attachment part.

On the other hand, to receive urine excreted from a female excretory organ, the attachment part may not be integrated with the introduction portion, but may be configured as a separate body having a shape highly adhesive to the human body, and urine may be transferred to the introduction portion and then received.

Further, for use in which the glans is inserted and positioned in the guiding space, even when it is configured that the receptacle portion of the container body is positioned above the introduction portion, urine can be guided upward by providing the check valve portion at the tip end of the guide portion to face the receptacle portion with the excretion direction upward, and urine can be guided to the receptacle portion by the urinary pressure of urine excreted.

On the other hand, to receive urine excreted from a female excretory organ, it is difficult to have the excretion direction upward, and accordingly the introduction portion and/or the guide portion are/is provided with a check valve structure, whereby urine can be guided to the receptacle portion.

Further, when a biological fluid to be received is highly viscous, such as menstrual blood and exudate, or has a high solid content, such as feces, it may be difficult to introduce and receive the biological fluid using the container body using merely the pressure during excretion and emission, and further, it may also be difficult to discharge the biological fluid after reception, so that the introduction portion and/or the guide portion and/or the receptacle portion and/or the outlet portion may be provided with a check valve structure.

The capacity of the receptacle portion of the container body of the biological fluid receptacle container according to the present invention can be determined depending upon an expected amount of a biological fluid to be received, and a large or small bulge part can be provided on a side of the receptacle portion as necessary, whereas by using a material having a low hardness and a high elongation, the receptacle portion can be configured in such a manner as to expand in accordance with a received amount of the biological fluid.

When the receptacle portion is provided with no bulge part and an angle and orientation of attachment are difficult to recognize from only the appearance of the container body, the angle and orientation of attachment may be indicated for the user by letters, symbols or the like displayed on the container body.

Note that it is desirable to provide the bulge part of the receptacle portion with the high elongation region designed for the maximum elongation during reception of a biological fluid, but when no bulge part is provided, the high elongation region can be provided at any position of the receptacle portion.

Any specific embodiment of the present invention may be suitable as long as the closure member of the biological fluid receptacle container according to the present invention closes an outlet port part of a container body comprising at least a hollow receptacle portion configured to receive a biological fluid and a hollow outlet portion configured to communicate with one side of this receptacle portion, including an outlet port part for discharging the biological fluid and a part to be fastened, and configured to discharge the biological fluid, the closure member together with the container body constituting the biological fluid receptacle container, in which the closure member includes: a fastening ring portion configured to surround the part to be fastened and elastically fasten the part to be fastened from the outside so as to close the outlet port part; and a pair of pressing portions arranged spaced apart from each other at corresponding positions on the outside of the fastening ring portion and in which leakage of urine can be reliably prevented and urine can be discharged through an easy operation.

Further, a material forming the closure member of the biological fluid receptacle container according to the present invention may be the same material as or a different material than a material constituting the container body, a flexible material may be used, and various thermoplastic elastomers, for example, urethane rubber or soft polyvinyl chloride, or various thermosetting elastomers, for example, silicone rubber may be used.

If the hardness of a material forming the closure member is set to be greater than the hardness of a material forming the container body, then deformation of the closure member can be reduced and reliable closure of the outlet portion can be achieved.

Any specific embodiment of the present invention may be suitable as long as the detachment prevention member is configured by integrally forming from a flexible material: a front covering portion including a semi-cylindrical front outer surface part and a semi-cylindrical front inner surface part, the front covering portion having a semi-cylindrical shape having a fixed thickness; and a back covering portion including a flat back outer surface part and a back inner surface part having a substantially V-shaped cross-sectional shape, the back covering portion having such a shape that a thickness thereof decreases toward a center, in which the detachment prevention member prevents such inconvenience that the penis slips out of the container body during use of the biological fluid receptacle container and does not hinder urination and urine collection even when being attached to the penis.

For example, it can be configured such that the penis is inserted and attached into the tubular detachment prevention member and then the container body is attached such that the introduction portion of the container body is expanded and opened to cover the detachment prevention member.

In a different configuration, it can be also configured such that the container body is attached to the penis and then the penis together with the container body are inserted and attached into the tubular detachment prevention member.

Further, a material forming the detachment prevention member according to the present invention may be the same material as or a different material than a material constituting the container body, a flexible material may be used, and various thermoplastic elastomers, for example, urethane rubber or soft polyvinyl chloride, or various thermosetting elastomers, for example, silicone rubber may be used.

Then, a preferred relationship between a thickness dimension of the container body and that of the detachment prevention member will be explained.

For the container body, the minimum value of the thickness dimension TA of the receptacle portion, i.e., the minimum thickness dimension thereof will be expressed as TAmin, the minimum value of the thickness dimension TI of the introduction portion, i.e., the minimum thickness dimension thereof as TImin, the minimum value of the thickness dimension TG of the guide portion, i.e., the minimum thickness dimension thereof as TGmin, and the minimum value of the thickness dimension TR of the detachment prevention member, i.e., the minimum thickness dimension thereof as TRmin.

Then, when the receptacle portion and the introduction portion of the container body are made of the same material, if the relational equation

$$TAmin < TGmin$$

is satisfied, then when the biological fluid receptacle container receives a biological fluid more than or equal to an initial capacity, the receptacle portion expands earlier than the guide portion, which can preferably prevent reception incapability caused by expansion of the guide portion while the check valve is closed due to an internal pressure of the receptacle portion.

Further, when the introduction portion and the guide portion of the container body are made of the same material, if the relational equation

$$TGmin < TImin$$

is satisfied, then when the biological fluid receptacle container receives a biological fluid more than or equal to an initial capacity, the guide portion expands earlier than the introduction portion, which can preferably prevent detachment of the container body caused by earlier expansion of the introduction portion than the guide portion and slipping of the penis out of the guide portion.

Moreover, when the receptacle portion and the introduction portion of the container body are made of the same material, if the relational equation

$$TAmin < TImin$$

is satisfied, then when the biological fluid receptacle container receives a biological fluid more than or equal to an initial capacity, regardless of a configuration of the support part of the guide portion, the receptacle portion expands earlier than the introduction portion, which can preferably prevent detachment of the container body caused by earlier expansion of the introduction portion than the receptacle portion and slipping of the penis out of the introduction portion.

Thus, when the receptacle portion, the introduction portion, and the guide portion of the container body are made of the same material, if the relational equation

$$TAmin < TGmin < TImin$$

is satisfied, then when the biological fluid receptacle container receives a biological fluid more than or equal to an initial capacity, the receptacle portion expands earlier than the guide portion, which can further preferably prevent reception incapability caused by expansion of the guide portion while the check valve is closed due to an internal pressure of the receptacle portion, the guide portion expands earlier than the introduction portion, which can further preferably prevent detachment of the container body caused by earlier expansion of the introduction portion than the guide portion and slipping of the penis out of the guide portion, and the receptacle portion expands earlier than the introduction portion, which can further preferably prevent detachment of the container body caused by earlier expansion of the introduction portion than the receptacle portion and slipping of the penis out of the introduction portion.

In a case of using the detachment prevention member together, when the container body and the tubular detachment prevention member are made of the same material, if the relational equation

$$TAmin < TGmin < TImin < TImin + TRmin$$

is satisfied, then detachment of the container body can be preferably prevented for the same reason as in the cases as described above.

Further, in a case of using the detachment prevention member together, when the hardness of a material constituting the detachment prevention member is k times that of a material constituting the container body, then the Young's modulus of the material constituting the detachment prevention member is also k times that of the material constituting the container body and the elongation per unit force is 1/k times, and accordingly, satisfying the relational expression

$$TAmin < TGmin < TImin + k \times TRmin$$

is preferable such that the container body can be prevented from detaching for the same reason as in the cases as described above.

When the foreskin accommodation window part is provided in the detachment prevention member, as long as the foreskin accommodation window part is configured to communicate from the front inner surface part to the front outer surface part, hold the loose foreskin within the foreskin accommodation window part when the detachment prevention member is attached to the penis, and prevent the foreskin from moving together with the foreskin accommodation window part to cover the glans, the foreskin accommodation window part may be formed as an opening part having a rounded rectangular shape, or further, a circular shape, an elliptical shape or any other shape.

Embodiment 1

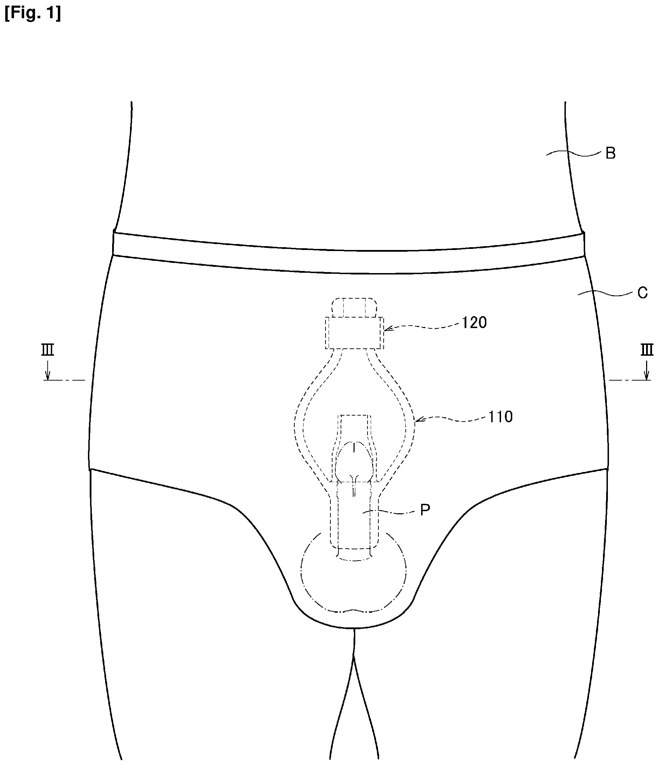

Hereinafter, a biological fluid receptacle container 100 according to a first embodiment of the present invention will be described with reference to fig. 1 to fig. 7B.

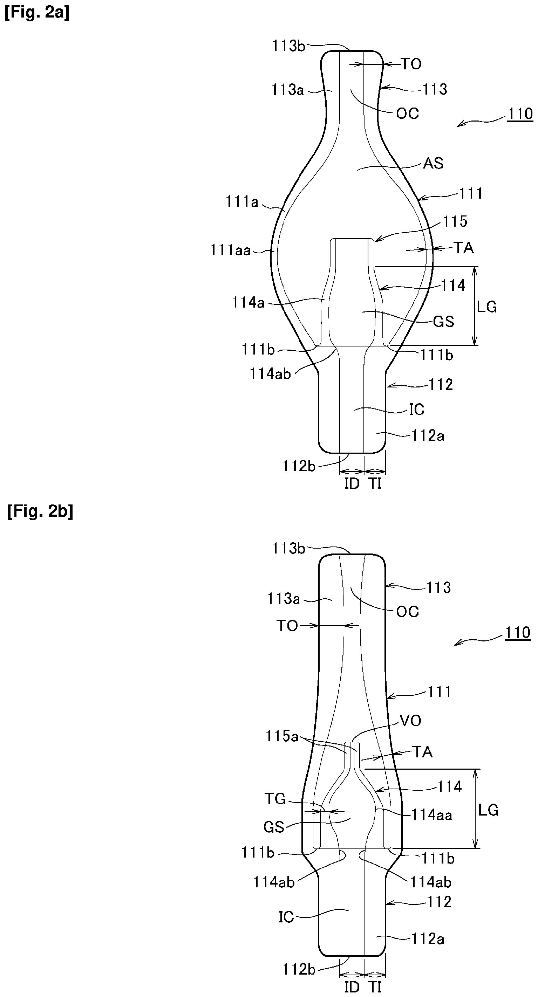

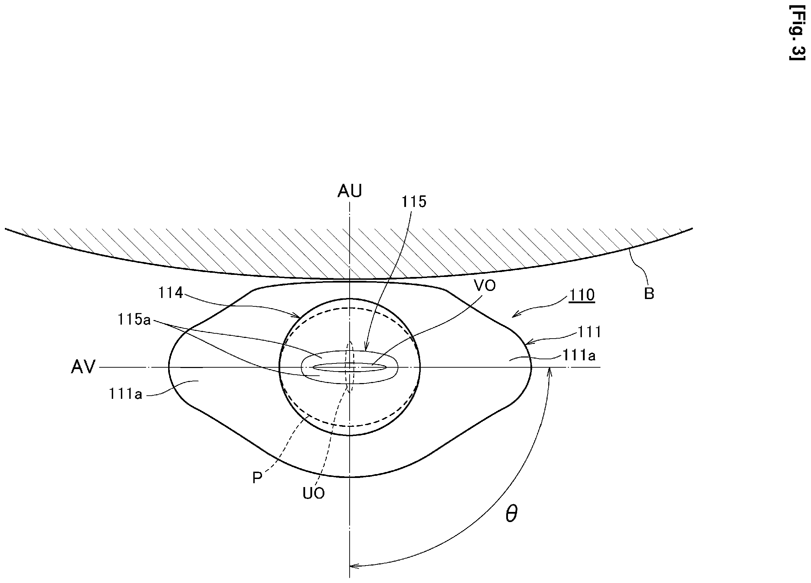

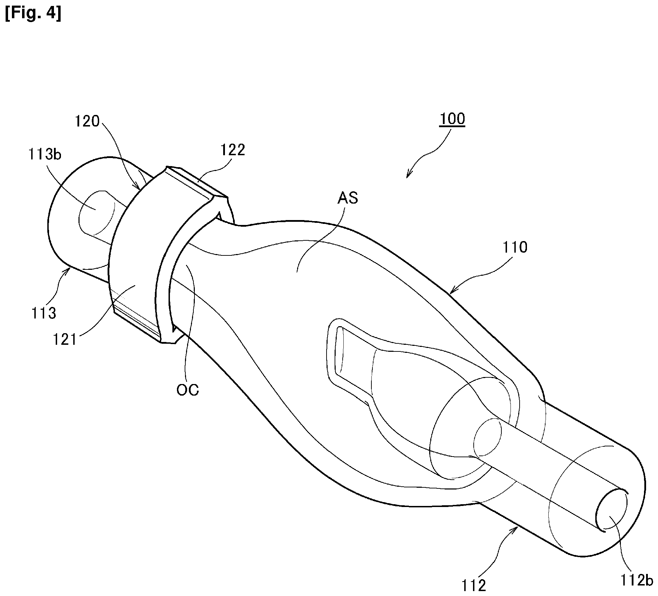

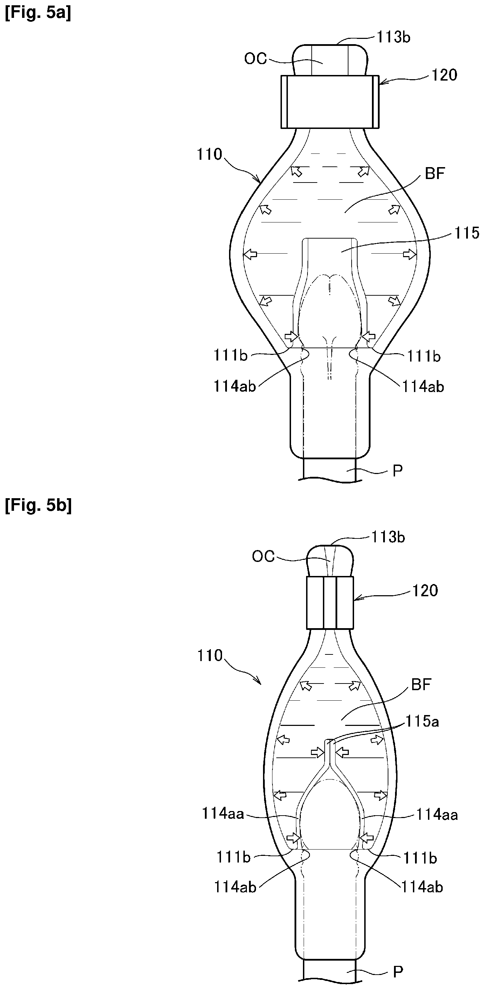

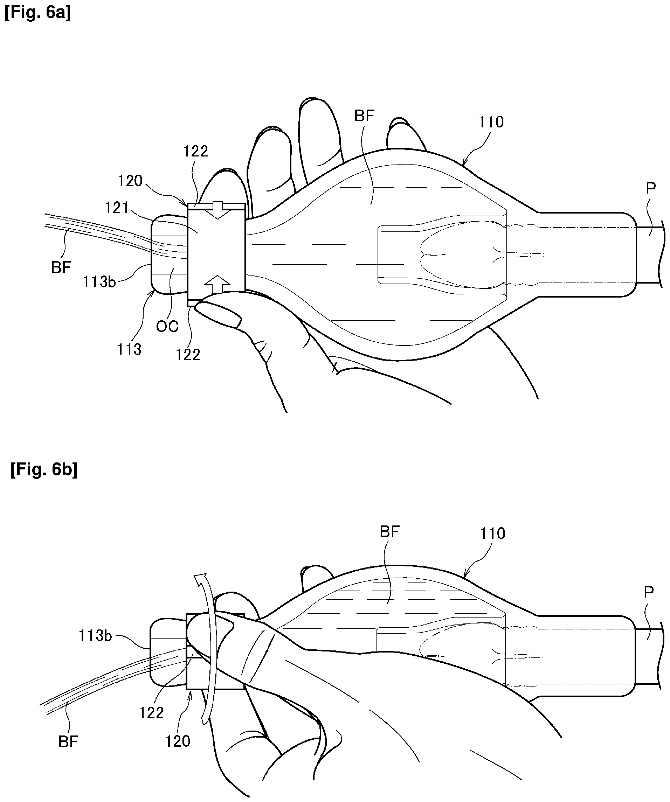

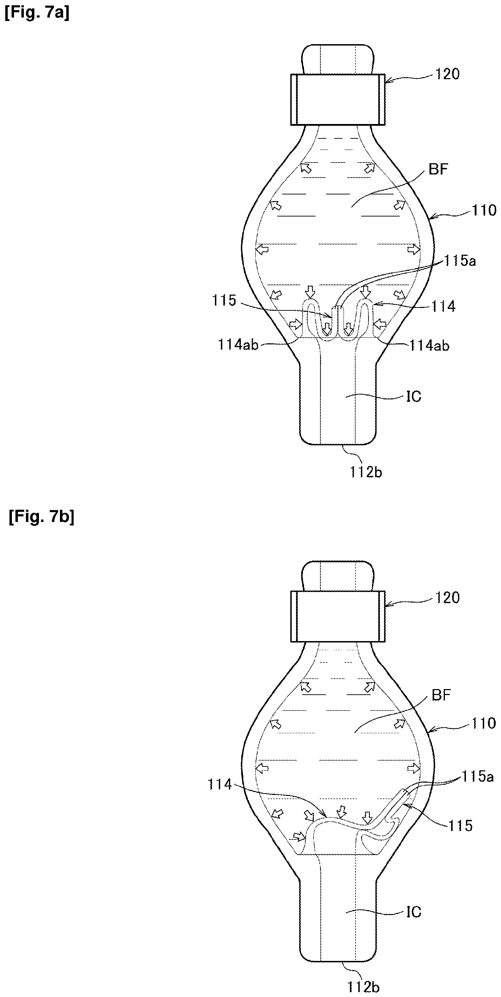

Then, fig. 1 is a schematic diagram illustrating a state in which a user wears on the penis a biological fluid receptacle container according to the first embodiment of the present invention, fig. 2A is a schematic front view illustrating a container body of the biological fluid receptacle container according to the first embodiment of the present invention, fig. 2B is a schematic side view illustrating the container body of the biological fluid receptacle container according to the first embodiment of the present invention, fig. 3 is a schematic diagram illustrating a downward view from the III-III cross section of fig. 1, fig. 4 is a schematic perspective view illustrating the biological fluid receptacle container according to the first embodiment of the present invention, fig. 5A is a schematic front view illustrating a state in which urine is received in a receptacle portion using the biological fluid receptacle container according to the first embodiment of the present invention, fig. 5B is a schematic side view illustrating a state in which urine is received in the receptacle portion using the biological fluid receptacle container according to the first embodiment of the present invention, fig. 6A is a schematic diagram illustrating a state of discharging a biological fluid received in the biological fluid receptacle container according to the first embodiment of the present invention, fig. 6B is a schematic diagram illustrating a state of adjusting a discharge direction of the biological fluid received in the biological fluid receptacle container according to the first embodiment of the present invention, fig. 7A is a schematic diagram illustrating a first example of a state in which the biological fluid receptacle container according to the first embodiment of the present invention has detached from the penis during use, and fig. 7B is a schematic diagram illustrating a second example of a state in which the biological fluid receptacle container according to the first embodiment of the present invention has detached from the penis during use.

First, a configuration of the biological fluid receptacle container 100 will be described.

The biological fluid receptacle container 100 includes a container body 110 and a closure member 120, and is used by inserting the penis P as an excretory organ of a human body B thereinto and being naturally held inside clothing C worn by the human body B as illustrated in fig. 1.

<Configuration of the container body>

As illustrated in fig. 2A to fig. 4, the container body 110 includes a receptacle portion 111, an introduction portion 112, an outlet portion 113, a guide portion 114, and a check valve portion 115, and each portion is formed integrally with one another from a material having a low hardness and a high elongation.

The low hardness and high elongation material used in the present embodiment is a thermoplastic elastomer, specifically, KURAIN (registered trademark) manufactured by Kuraray Trading Co., Ltd. which is made of a compound containing at least five times paraffin oils in a styrene-based thermoplastic elastomer (TPS: Thermoplastic Styrene Elastomer) and is a material having a hardness of 10 degrees or less and an elongation of 2000% or more.

<Configuration of the receptacle portion>

The receptacle portion 111 has such a hollow shape as to form a receiving space AS for receiving urine BF as a biological fluid, has such a flat shape as to have a bulge part 111a which bulges in a lateral direction, and has a receiving space bottom part 111b which forms the lowest position of the receiving space AS when in use.

A thickness dimension TA of the receptacle portion 111 is different depending upon regions, and a thickness dimension at the bulge part 111a has a smaller value than the thickness dimension TA at the other parts. Accordingly, a thin-walled region 111aa at which the thickness dimension TA of the receptacle portion 111 is the smallest is positioned in the bulge part 111a.

In the present embodiment, the thickness dimension TA of the receptacle portion 111 at the thin-walled region 111aa is about 2 mm.

In the present embodiment, the receptacle portion 111, the introduction portion 112, the outlet portion 113, the guide portion 114, and the check valve portion 115 are integrally formed from a material having a low hardness and a high elongation, so that the thin-walled region 111aa has the highest elongation, and accordingly the thin-walled region 111aa corresponds to a high elongation region.

<Configuration of the introduction portion>

The introduction portion 112 includes an introduction port part 112b for introducing the urine BF, has such a hollow shape as to have an introduction channel IC formed in the interior of a tubular attachment part 112a, and communicates with one side of the receptacle portion 111.

In the present embodiment, the introduction channel IC has a circular cross-sectional shape for use by inserting into the introduction channel IC the penis P of a user.

In the present embodiment, a thickness dimension TI of the introduction portion is about 5 mm, and an inner diameter dimension ID of the introduction channel is about 10 mm.

<Configuration of the outlet portion>

The outlet portion 113 includes an outlet port part 113b for discharging the urine BF, has such a hollow shape as to have an outlet channel OC formed in the interior of a part to be fastened 113a, and communicates with the other side of the receptacle portion 111.

In the present embodiment, a thickness dimension TO of the outlet portion is different depending upon regions and is about 3 mm or more and about 5 mm or less.

<Configuration of the guide portion 114>

The guide portion 114 is formed at a region at which the receptacle portion 111 and the introduction portion 112 communicate with each other to have such a hollow shape as to protrude toward the receiving space AS in the receptacle portion 111 and guides the urine BF in a guiding space GS into the receptacle portion 111.

A thickness dimension TG of the guide portion 114 is different depending upon regions and, in the present embodiment, is about 3 mm or more and about 5 mm or less.

The guide portion 114 forms the guiding space GS having a length dimension LG of the guide portion by a support part 114a for supporting the check valve portion 115.

In the present embodiment, the length dimension LG of the guide portion is about 20 mm.

In the present embodiment, for use by inserting and disposing the glans G of a user into the guiding space GS, a large diameter region 114aa is formed substantially at the center of the support portion 114a in a length direction which forms the guiding space GS, and a small diameter region 114ab is formed at a position corresponding to the receiving space bottom part 111b of the receptacle portion 111.

In other words, the receiving space bottom part 111b of the receptacle portion 111 is arranged at a position corresponding to the small diameter region 114ab, and this small diameter region 114ab is positioned closer to the introduction portion 112 than the large diameter region 114aa of the support part 114a of the guide portion 114.

<Configuration of the check valve portion>

The check valve portion 115 is formed at a tip end of the guide portion 114 in such a manner as to protrude toward the receptacle portion 111 in order to prevent backflow of the urine BF and includes a pair of valve membrane parts 115a arranged facing each other and toward the receptacle portion 111 at respective positions close to each other in a manner separate from each other.

The pair of valve membrane parts 115a are arranged facing each other at respective positions close to each other in a manner separate from each other, so that when the check valve portion 115 is in a state in which no urine BF is received in the receptacle portion 111, a valve opening VO is in an open state in an oval slit shape.

As described above, the container body 110 constituting the biological fluid receptacle container 100 is configured by integrally forming from a thermoplastic styrenic elastomer having a low hardness and a high elongation: the hollow receptacle portion 111 forming the receiving space AS configured to receive the urine BF; the hollow introduction portion 112 configured to communicate with one side of this receptacle portion 111, including the introduction port part 112b for introducing the urine BF, and forming the introduction channel IC; the hollow outlet portion 113 configured to communicate with the other side of the receptacle portion 111, including the outlet port part 113b for discharging the urine BF, and forming the outlet channel OC; the hollow guide portion 114 formed at a region at which the receptacle portion 111 and the introduction portion 112 communicate with each other in such a manner as to protrude toward the interior of the receptacle portion 111 and configured to guide the urine BF into the receptacle portion 111; and the check valve portion 115 formed at the guide portion 114 and configured to prevent backflow of the urine BF.

<Configuration of the closure member>

As illustrated in fig. 4, the closure member 120 includes a fastening ring portion 121 configured to surround the part to be fastened 113a of the container body 110 constituting the biological fluid receptacle container 100 and elastically fasten the part to be fastened 113a from the outside so as to close the outlet port part 113b and a pair of pressing portions 122 arranged spaced apart from each other at corresponding positions on the outside of this fastening ring portion 121, and is integrally formed from a flexible material.

<Process of use>

Next, a process of using the biological fluid receptacle container 100 will be described with reference to fig. 1 to fig. 6B.

The introduction portion 112 of the container body 110 is integrally formed from a thermoplastic elastomer having a low hardness and a high elongation, so that a user can easily expand and open the introduction port part 112b by applying a force in such a manner as to turn over the attachment part 112a using both hands.

In this case, first, the outlet portion 113 is temporarily closed by the closure member 120, then the introduction port part 112b is expanded and opened and the penis P of a user is inserted into the introduction portion 112, and subsequently the hands expanding and holding the attachment part 112a are released, thereby attaching the attachment part 112a to the penis P.

Note that in inserting the penis P into the introduction portion 112, if the glans G is inserted to such a position as to be accommodated well in the guide portion 114, adjustment of an attachment position is unnecessary.

Further, squeezing the receptacle portion 111 from the front and back to remove the air from the receiving space AS at this stage allows the container body 110 to prevent from being bulky and the receiving space AS to be efficiently used for reception.

Then, as illustrated in fig. 3, the container body 110 according to the present embodiment is configured such that a major axis direction AV of an oval valve opening VO formed by the pair of valve membrane parts 115a constituting the check valve portion 115 coincides with a lateral direction of the container body 110.

Accordingly, in attaching the container body 110 to the penis P, it is desirable to determine an angle of attachment such that the lateral bulge parts 111a of the receptacle portion 111 substantially correspond to a lateral direction of a wearer, that is, such that the major axis direction AV of the valve opening VO substantially corresponds to a lateral direction of a wearer.

This is because even when the guide portion 114 formed from a thermoplastic elastomer having a low hardness and a high elongation deforms during attachment and the check valve portion 115 is displaced from the center, which causes the external urethral orifice UO and the valve opening VO to be offset from each other, then a major axis direction AU of the external urethral orifice UO of the penis P and the major axis direction AV of the valve opening VO are substantially perpendicular to each other and an angle of deviation $\theta$ is about 90 degrees, which makes it easy for a part of the external urethral orifice UO to overlap with the valve opening VO and thus makes it difficult for the external urethral orifice UO to be blocked.

Then, as illustrated in fig. 4, by securely fitting the part to be fastened 113a of the outlet portion 113 of the container body 110 in such a manner as to be surrounded by the closure member 120, a restoring force of the elastically deformed fastening ring portion 121 causes the closure member 120 to fasten the part to be fastened 113a from the outside, thereby closing the outlet channel OC near the outlet port part 113b, which results in an attachment state as illustrated in fig. 1.

When urination is carried out in a state in which the biological fluid receptacle container 100 is attached to the penis P as illustrated in fig. 1, at a stage of beginning of urination, the valve opening VO formed by the pair of valve membrane parts 115a of the check valve portion 115 is in an open state, so that even when the urine pressure is low, the urine BF is reliably guided into the receptacle portion 111 and received in the receiving space AS.

On the other hand, when the urine pressure is high, the pair of valve membrane parts 115a of the check valve portion 115 stretch and an opening area of the valve opening VO increases, so that the urine is quickly guided into the receptacle portion 111 and received in the receiving space AS.

When urination continues and an amount of the received urine BF is more than or equal to an initial capacity of the receiving space AS, the receptacle portion 111 begins to expand as illustrated in fig. 5A and fig. 5B.

In the present embodiment, the receptacle portion 111, the introduction portion 112, the outlet portion 113, the guide portion 114, and the check valve portion 115 are integrally formed from a thermoplastic elastomer having a low hardness and a high elongation, so that the thin-walled region 111aa as illustrated in fig. 2 has the highest elongation and the thin-walled region 111aa as a high elongation region stretches and expands earlier than the other regions.

Note that the thin-walled region 111aa of the receptacle portion 111 has a higher elongation than any region of the introduction portion 112, the outlet portion 113, and the guide portion 114.

When the receptacle portion 111 expands, an internal pressure of the urine BF increases and exerts a force on a region in contact with the urine BF, and, as indicated by arrows in fig. 5A and fig. 5B, this force acts on the receptacle portion 111 in such a manner as to push and expand an inner wall thereof outward and acts on the guide portion 114 and the check valve portion 115 in such a manner as to squeeze the same inward.

Thus, the valve opening VO of the check valve portion 115 is in an open state when no urine BF is contained in the receptacle portion 111, whereas in a state in which the urine BF is contained, the pair of valve membrane parts 115a are pushed from the outside by an internal pressure and made to come into contact with each other so as to close the valve opening VO, which prevents the urine BF from flowing back from the receptacle portion 111 to the introduction portion 112.

Further, this internal pressure acts at the receiving space bottom part 111b in such a manner as to squeeze inward the small diameter region 114ab of the support part 114a of the guide portion 114, so that the small diameter region 114ab fits into the annular groove of the penis P, which prevents the container body 110 from detaching due to slipping of the penis P out of the introduction portion 112 caused by an increase in an internal pressure.

As illustrated in fig. 6A, to discharge the urine BF contained in the receptacle portion 111, an operation is carried out such that while the biological fluid receptacle container 100 is attached to the penis P, the pair of pressing portions 122 of the closure member 120 are held in one hand and a force is applied in such a manner as to pinch the pair of pressing portions 122 inward with the index finger and thumb.

This operation allows the fastening ring portion 121 surrounding and fastening the part to be fastened 113a of the outlet portion 113 from the outside to be expanded due to an elasticity, thereby releasing the outlet channel OC in the interior of the outlet portion 113 formed from a thermoplastic elastomer having a low hardness and a high elongation, and accordingly the urine BF is discharged from the outlet port part 113b.

If a discharge direction of the urine BF is unstable, an operation is carried out as illustrated in fig. 6B such that the pair of pressing parts 122 of the closure member 120 is held in one hand and, while a force is applied in such a manner as to pinch the pair of pressing portions 122 inward with the index finger and thumb, further the pair of pressing parts 122 are twisted in either direction around a longitudinal direction of the outlet channel OC in the interior of the outlet portion 113.

This operation allows the outlet portion 113 formed from a thermoplastic elastomer having a low hardness and a high elongation to be deformed in a twisted manner, and accordingly spiral irregularities are caused on an inner wall surface of the outlet channel OC in the interior of the outlet portion 113, which stabilizes a discharge direction of the urine BF.

Next, a case in which the biological fluid receptacle container 100 according to the present embodiment unexpectedly detaches from the penis P for some reason when in use will be described with reference to fig. 7A and fig. 7B.

When the biological fluid receptacle container 100 unexpectedly detaches from the penis P for some reason, the glans inserted and placed in the guiding space GS is then no longer present and consequently, on the support part 114a of the guide portion 114 which forms the guiding space GS, a pushing force due to an internal pressure of the urine BF contained therein acts into a direction of the introduction channel IC in the interior of the introduction portion 112.

The length dimension LG of the guide portion is configured to be larger than half the inner diameter dimension ID of the introduction channel IC formed in the introduction portion 112 such that if this pushing force acts evenly, the entirety of the guide portion 114 deforms symmetrically to block the introduction channel IC as illustrated in fig. 7A.

In this case, the valve opening VO formed by the pair of valve membrane parts 115a remains closed due to an internal pressure of the urine BF, so that the urine BF does not flow back from the receptacle portion 111 to the introduction channel IC, which can prevent the urine BF from leaking out of the introduction port part 112b.

On the other hand, if the pushing force acts unevenly, the entirety of the guide portion 114 deforms asymmetrically to block the introduction channel IC as illustrated in fig. 7B.

Also in this case, the valve opening VO formed by the pair of valve membrane parts 115a remains closed due to an internal pressure of the urine BF, so that the urine BF does not flow back from the receptacle portion 111 to the introduction channel IC, which can prevent the urine BF from leaking out of the introduction port part 112b.


Embodiment 2

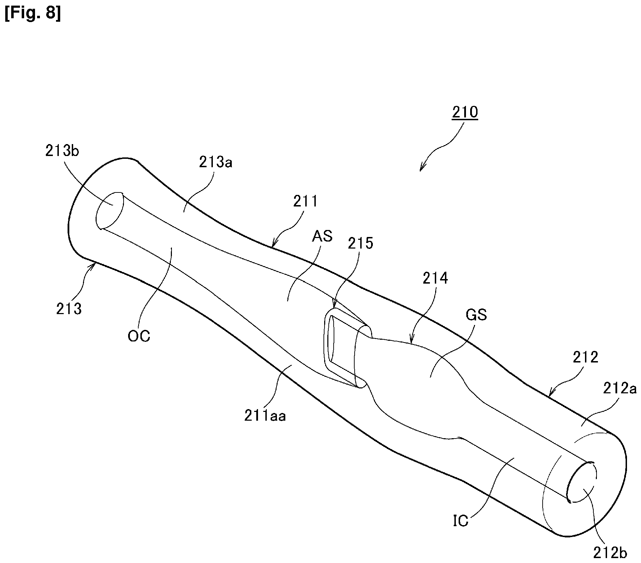

Hereinafter, a container body 210 according to a second embodiment of the present invention will be described with reference to fig. 8. Then, fig. 8 is a schematic perspective view illustrating a container body according to the second embodiment of the present invention.

The container body 210 includes a receptacle portion 211, an introduction portion 212, an outlet portion 213, a guide portion 214, and a check valve portion 215, and each portion is formed integrally with one another from a material having a low hardness and a high elongation.

The container body 210 has a substantially cylindrical shape in which the receptacle portion 211 is not provided with a bulge part.

Further, the container body 210 is configured such that the guide portion 214 is positioned between the receptacle portion 211 and the introduction portion 212 to communicate with one another.

Although the guide portion 214 in the present embodiment forms the guiding space GS, the support part which is an independent structure in the first embodiment is integrated with the receptacle portion 211 in a thickness direction in the present embodiment, so that the guide portion 214 is not configured to protrude toward the receiving space AS in the receptacle portion 211 and a thickness dimension of the guide portion 214 substantially constitutes a part of a thickness dimension of the receptacle portion 211.

The container body 210 has the configurational differences as described above in comparison to the container body 110 according to the first embodiment, so that although the container body 210 has a slightly smaller capacity than the container body 110 according to the first embodiment, the container body 210 is excellent in portability and can be suitably used when an amount of urine BF as a biological fluid to be contained is small.

Apart from the operations and effects relating to the above differences, the container body 210 exhibits through similar operations similar effects to those by the container body 110 according to the first embodiment.

Embodiment 3

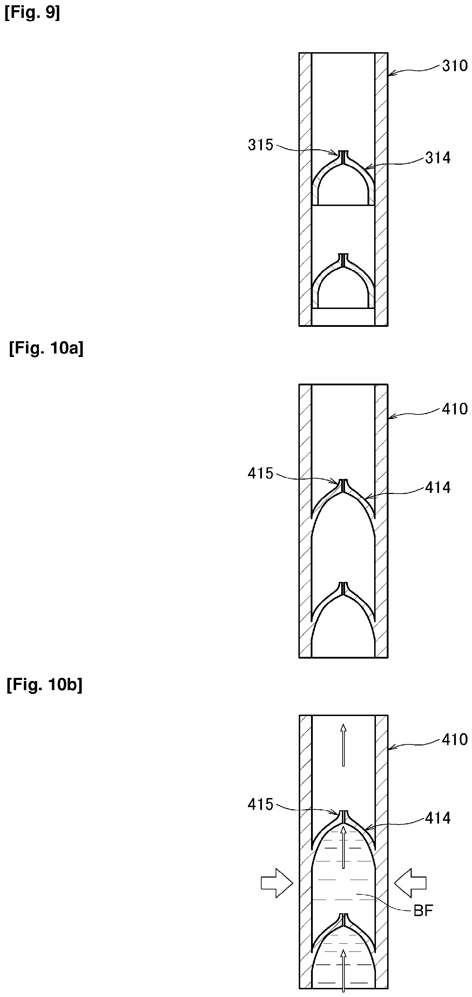

Hereinafter, a container body 310 according to a third embodiment of the present invention will be described with reference to fig. 9. Then, fig. 9 is a schematic diagram illustrating a state in which a receptacle portion according to the third embodiment of the present invention includes a plurality of sets of guide portions and check valve portions.

The container body 310 includes a plurality of sets of guide portions 314 and check valve portions 315 in the interior of the hollow container body 310 in fig. 9, and the container body 310 is configured such that the plurality of sets of guide portions 314 and check valve portions 315 integrally formed from a thermoplastic elastomer having a low hardness and a high elongation are prepared, and then inserted and fixed in the container body 310 formed from a thermoplastic elastomer having a low hardness and a high elongation.

Embodiment 4

Hereinafter, a container body 410 according to a fourth embodiment of the present invention will be described with reference to fig. 10A and fig. 10B. Then, Fig. 10A is a schematic diagram illustrating a state in which a receptacle portion according to the fourth embodiment of the present invention includes a plurality of sets of guide portions and check valve portions, and fig. 10B is a schematic diagram illustrating a state in which a biological fluid is moved upward in the receptacle portion illustrated in fig. 10A.

As illustrated in fig. 10A, the container body 410 includes a plurality of sets of guide portions 414 and check valve portions 415 in the interior of the hollow container body 410, and it is configured that the container body 410, the guide portions 414, and the check valve portions 415 are integrally formed from a thermoplastic elastomer having a low hardness and a high elongation.

The container body 410 includes the plurality of sets of guide portions 414 and check valve portions 415 in the interior of the hollow container body 410, so that the container body 410 is pressed from the exterior as illustrated in fig. 10B, whereby the urine BF as a biological fluid received in the interior of the container body 410 can be sequentially moved upward against gravity.

[Reference Example 1]

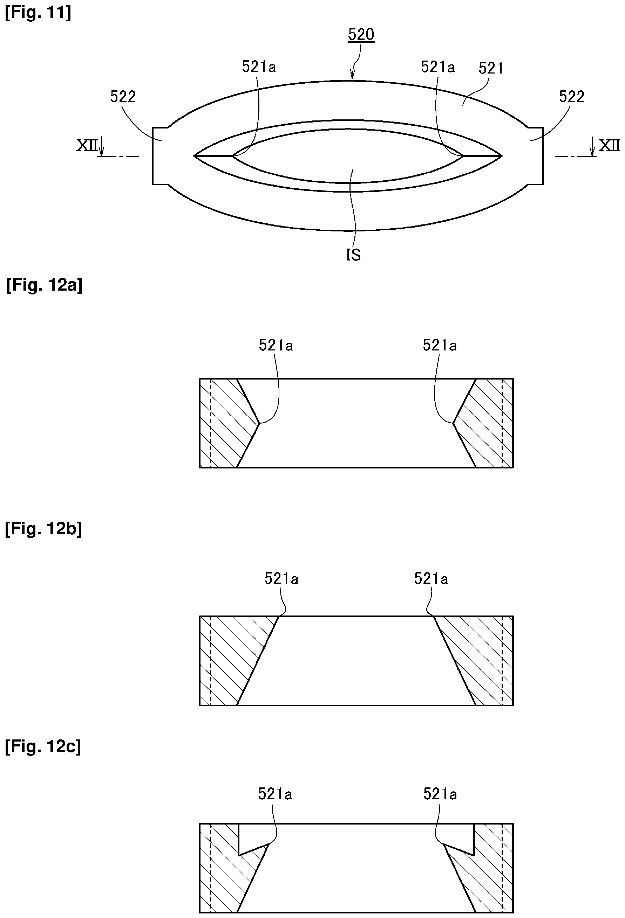

Next, a closure member 520 according to a first reference example of the present invention will be described with reference to fig. 11 to fig. 12C. Then, fig. 11 is a diagram illustrating a closure member according to the first reference example of the present invention, fig. 12A is a schematic cross-sectional view illustrating a first example of a cross section of fig. 11 along XII-XII, fig. 12B is a schematic cross-sectional view illustrating a second example of a cross section of fig. 11 along XII-XII, and fig. 12C is a schematic cross-sectional view illustrating a third example of a cross section of fig. 11 along XII-XII.

As illustrated in fig. 11, a closure member 520 includes a fastening ring portion 521 forming a fastening insertion space IS and a pair of pressing portions 522 arranged in a manner separate from each other at corresponding positions on the outside of this fastening ring portion 521, and further includes an inner-ring protrusion portion 521a at positions on the inside of the fastening ring portion 521 corresponding to the pressing portions 522, and the closure member 520 is integrally formed from a flexible material.

As illustrated in fig. 12A, a first example of a shape of the inner-ring protrusion portion 521a is a shape protruding in a V-shaped manner toward the center of the fastening ring portion 521 in a thickness direction.

When the inner-ring protrusion portion 521a has such a shape, the part to be fastened inserted in the fastening insertion space IS can be tightly fastened at a center region, so that the outlet channel OC of the container body can be reliably closed.

As illustrated in fig. 12B, a second example of a shape of the inner-ring protrusion portion 521a is a shape configured to narrow the fastening insertion space IS toward one side of the fastening ring portion 521 such that an outlet side of the fastening ring portion 521 is narrower than an inlet side thereof.

When the inner ring protrusion portion 521a has such a shape, the outlet port part of the container body can be closed after completion of an operation of discharging the urine BF as a biological fluid such that an opening area of the outlet port part of the container body is small, so that an amount of the biological fluid remaining into the outlet channel OC can be reduced.

As illustrated in fig. 12C, a third example of a shape of the inner-ring protrusion portion 521a is a shape protruding at an acute angle toward the outlet side of the fastening ring portion 521.

When the inner-ring protrusion portion 521a has such a shape, it is possible to provide the closure member which makes it easy to insert the outlet portion of the container body into the fastening insertion space IS and is less likely to detach.

[Reference Example 2]

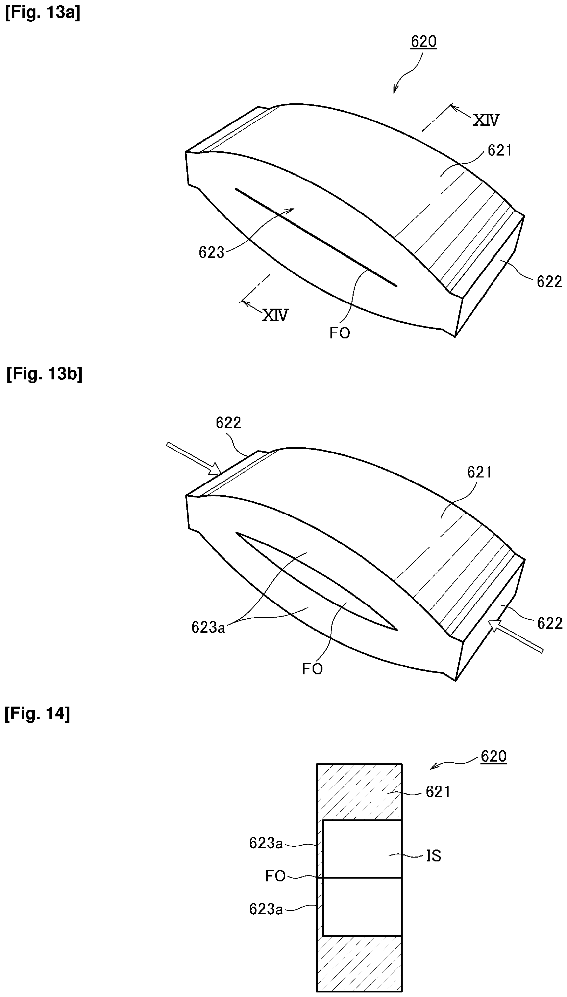

Next, a closure member 620 according to a second reference example of the present invention will be described with reference to fig. 13A to fig. 14. Then, fig. 13A is a diagram illustrating a closure member according to a second reference example of the present invention, fig. 13B is a diagram illustrating a state in which a closure valve portion of the closure member illustrated in fig. 13A is open, and fig. 14 is a cross-sectional view of fig. 13A along XIV-XIV.

As illustrated in fig. 13A, the closure member 620 according to the second reference example of the present invention includes a fastening ring portion 621 and a pair of pressing portions 622 arranged in a manner separate from each other at corresponding positions on the outside of this fastening ring portion 621, and further includes a closure member valve portion 623 at one of opening positions of the fastening ring portion 621, and the closure member 620 is integrally formed from a flexible material.

The closure member 620 is closed at one of the opening positions by means of the closure member valve portion 623 and is configured to be used by crowing the outlet portion so as to cover the outlet port part of the container body.

The closure member valve portion 623 includes a pair of closure member valve membrane parts 623a to form a closure member valve opening FO, and in a normal state, the pair of closure member valve membrane parts 623a are in close contact with each other such that the closure member valve opening FO is closed.

To discharge the urine BF contained in the receptacle container closed by crowing with the closure member 620, as illustrated in fig. 13B, the pair of pressing portions 622 are pressed inward, whereby the pair of closure member valve membrane parts 623a are separated from each other and thus the closure member valve opening FO becomes open.

When pressing the pressing portion 622 is stopped, the closure member valve opening FO becomes closed and thus discharge of the urine BF is stopped.

[Reference Example 3]

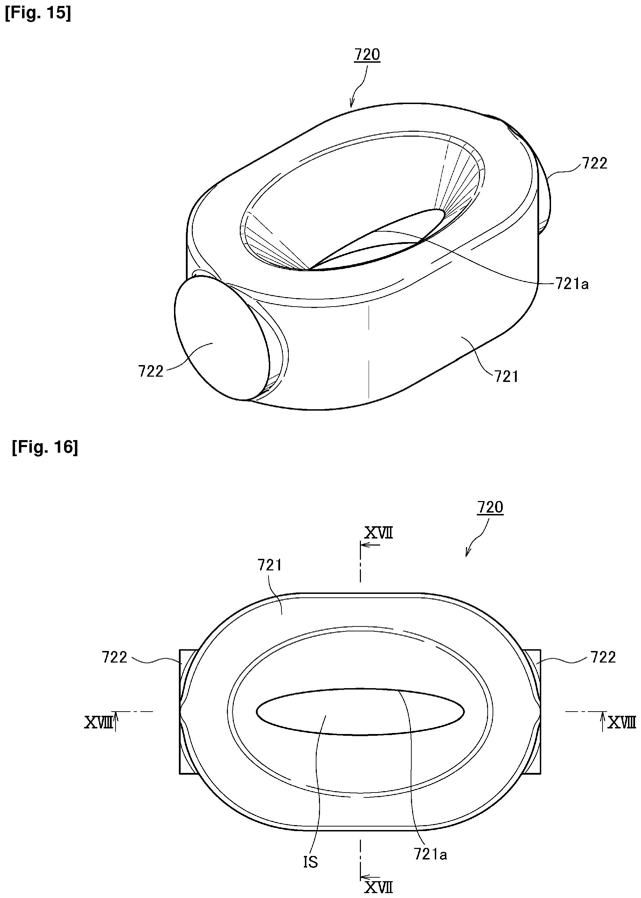

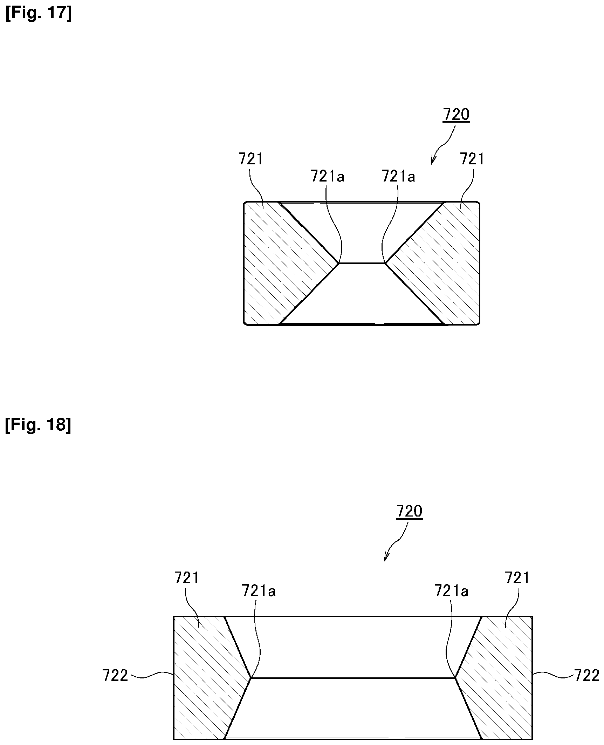

Next, a closure member 720 according to a third reference example of the present invention will be described with reference to fig. 15 to fig. 18. Then, fig. 15 is a diagram illustrating a closure member according to the third reference example of the present invention, fig. 16 is a plan view of the closure member illustrated in fig. 15, fig. 17 is a cross-sectional view of fig. 16 along XVII-XVII, and fig. 18 is a cross-sectional view of fig. 16 along XVIII-XVIII.

As illustrated in fig. 15 and fig. 16, the closure member 720 according to the present reference example includes a fastening ring portion 721 forming the fastening insertion space IS and a pair of pressing portions 722 arranged in a manner separate from each other at corresponding positions on the outside of this fastening ring portion 721, an inner-ring protrusion portion 721a is formed on an inner side of the fastening ring portion 721 around the entire circumference, and the closure member 720 is integrally formed from a flexible material.

As illustrated in fig. 17 and fig. 18, a shape of the inner-ring protrusion portion 721a is configured to protrude in a V-shaped manner toward the center of the fastening ring portion 721 in a thickness direction.

The inner-ring protrusion portion 721a has such a shape, whereby the part to be fastened inserted in the fastening insertion space IS can be tightly fastened at a center region, so that the outlet channel OC of the container body can be reliably closed.

[Reference Example 4]

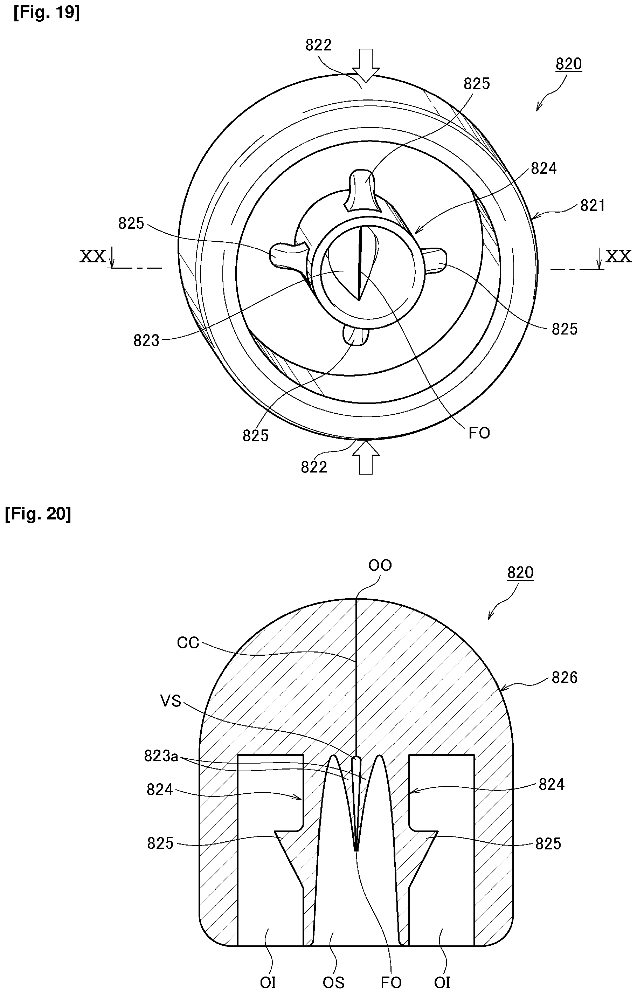

Next, a closure member 820 according to a fourth reference example of the present invention will be described with reference to fig. 19 and fig. 20. Then, fig. 19 is a diagram illustrating a closure member according to the fourth reference example of the present invention, and fig. 20 is a schematic cross-sectional view of fig. 19 along XX-XX.

As illustrated in fig. 19 and fig. 20, the closure member 820 according to the present reference example includes an annular fastening ring portion 821, a pressing portion 822, a closure member valve portion 823, an outlet channel insertion portion 824, a stopper protrusion portion 825, and a closure lid portion 826 and is integrally formed from a flexible material.

The closure member 820 includes an annular fastening ring portion 821, a pair of pressing portions 822 arranged in a manner separate from each other at corresponding positions on the outside of the fastening ring portion 821, and a dome-shaped closure lid portion 826 configured to block an upper portion of the fastening ring portion 821.

Further, at a lower portion of the closure lid portion 826 inside the fastening ring portion 821, the cylindrical outlet channel insertion portion 824 hangs down to form an outlet channel blocking space OS inside the cylinder and form an annular outlet portion insertion space OI outside the outlet channel insertion portion 824 and inside the fastening ring portion 821.

On a cylindrical outer surface of the outlet channel insertion portion 824, the four stopper protrusion portions 825 are formed at symmetrical respective positions in such a manner as to protrude toward the outlet portion insertion space OI.

In the interior of the outlet channel insertion portion 824, the closure member valve portion 823 consisting of a pair of closure member valve membrane parts 823a hangs down from the lower portion of the closure lid portion 826, thereby forming a check valve structure.

The pair of closure member valve membrane parts 823a each have both side end parts connected to an inner surface of the outlet channel insertion portion 824 so as to maintain watertightness, while each having straight lower end parts in close contact with each other so as to form the slit-shaped closure member valve opening FO in a closed state.

Moreover, in the closure member valve portion 823, an intervalve space VS is formed between the pair of closure member valve membrane parts 823a, and further, a closure member outlet channel CC is formed as a band-shaped space in a closed state which communicates from the intervalve space VS to the slit-shaped closure member outlet port OO in a closed state at the top of the closure lid portion 826.

Note that when the pressing portions 822 are not pressed inward from both sides, the closure member valve opening FO, the closure member outlet channel CC, and the closure member outlet port OO are in a closed state due to an elasticity of the material as described above.

When the closure member 820 is used together with the container body, the cylindrical outlet portion of the container body is inserted into the outlet portion insertion space OI, whereby the outlet channel insertion portion 824 is inserted into the outlet channel OC of the container body and thus the outlet channel is blocked.

In this case, the stopper protrusion portions 825 bite into a cylindrical outlet portion inner surface configured to form the discharge path OC of the container body, thereby preventing the closure member 820 from detaching from the container body.

In this state, the closure member valve opening FO, the closure member outlet channel CC, and the closure member outlet port OO are in a closed state, so that the urine BF as a biological fluid that has moved into the outlet channel blocking space OS does not leak.

To discharge the urine BF, the pressing portions 822 are pressed inward from both sides to deform the closure lid portion 826 and the closure member valve portion 823, so as to allow the closure member valve opening FO, the closure member outlet channel CC, and the closure member outlet port OO to become open, thereby discharging the urine BF from the closure member outlet port OO.

When pressing the pressing portion 822 is stopped, the closure member valve opening FO, the closure member outlet channel CC, and the closure member outlet port OO become closed and thus discharge of the urine BF is stopped.

Embodiment 5

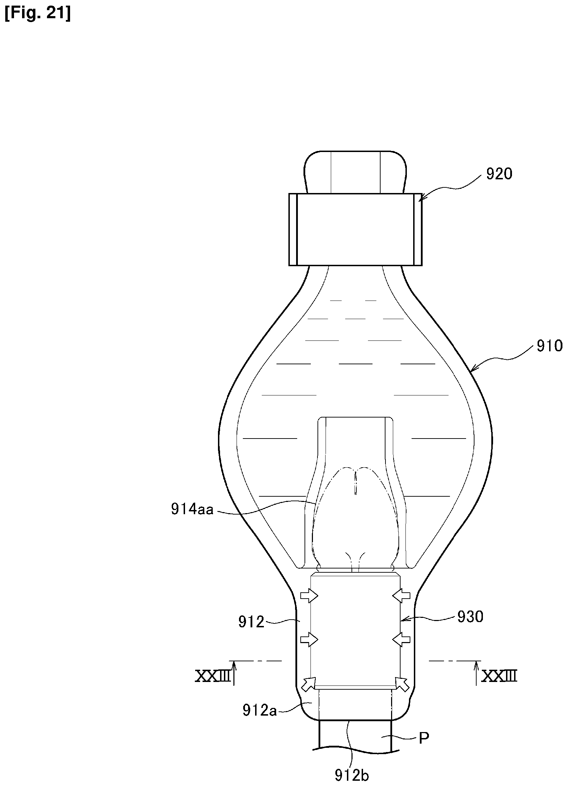

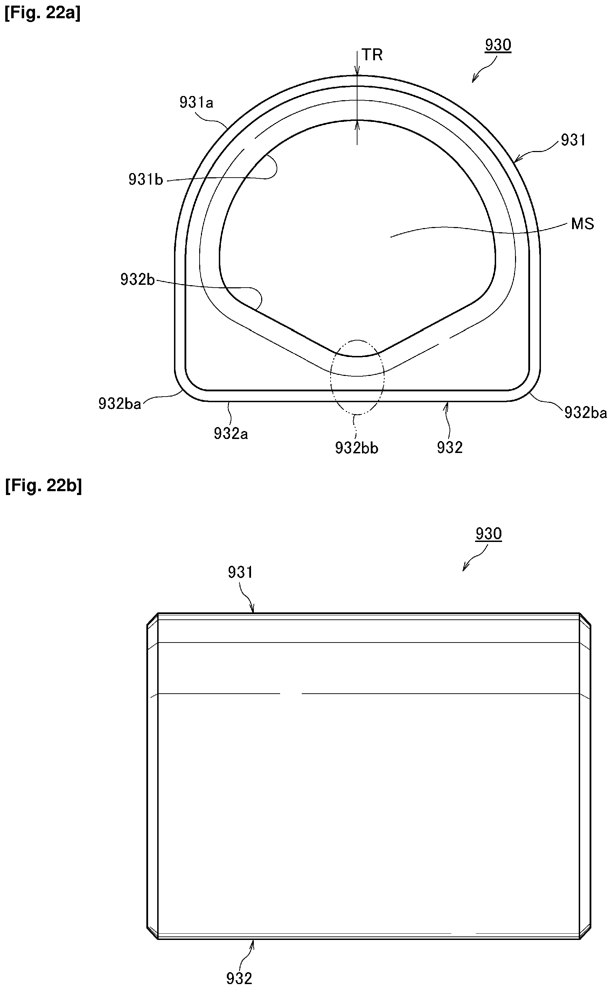

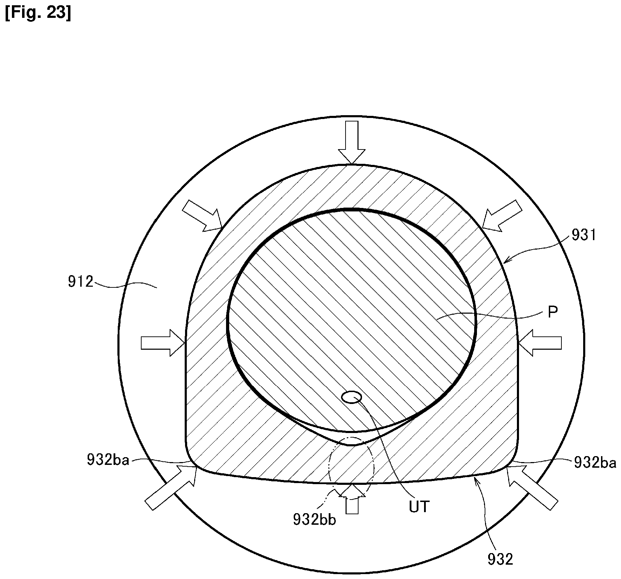

Next, a detachment prevention member 930 according to a fifth embodiment of the present invention will be described with reference to fig. 21 to fig. 23. Then, fig. 21 is a schematic diagram illustrating a state in which a user attaches a detachment prevention member according to the fifth embodiment of the present invention to the penis and then attaches a biological fluid receptacle container, fig. 22A is a schematic diagram of the detachment prevention member illustrated in fig. 21 as seen from an insertion direction, fig. 22B is a schematic side view of the detachment prevention member illustrated in fig. 21, and fig. 23 is a schematic cross-sectional view of fig. 21 along XXIII-XXIII.

The detachment prevention member 930 can be used as an accessory together with a biological fluid receptacle container including a container body 910 and a closure member 920, and the container body 910, the closure member 920, and the detachment prevention member 930 can constitute a biological fluid receptacle system.

Note that the container body 910 and the closure member 920 are similar in configuration to the container body 110 and the closure member 120 according to the first embodiment.

As illustrated in fig. 21, the detachment prevention member 930 is configured to be used to prevent the container body 910 from detaching by being attached to the penis P in advance to insert the penis P into the container body 910.

As illustrated in fig. 22A and fig. 22B, the detachment prevention member 930 is integrally formed by a front covering portion 931 and a back covering portion 932 from a flexible material.

The front covering portion 931 includes a semi-cylindrical front outer surface part 931a and a semi-cylindrical front inner surface part 931b and has a semi-cylindrical shape having a fixed thickness.

The back covering portion 932 includes a flat back outer surface part 932a and a back inner surface part 932b having a substantially V-shaped cross-sectional shape and has such a shape that a thickness thereof decreases toward the center, and the back covering portion 932 has a protrusion region 932ba configured to protrude outward at both left and right ends as seen from the insertion direction and a center region 932bb having the smallest thickness.

To use the detachment prevention member 930, first, while the foreskin F of the penis P is lowered to the glans ring (annular groove), the penis P is inserted into an attachment insertion space MS of the detachment prevention member 930 and attached thereto.

In this state, the back covering portion 932 includes the back inner surface part 932b having a substantially V-shaped cross-sectional shape and has such a shape that a thickness thereof decreases toward the center, so that at the center region 932bb, a pressure is relieved on the urethra UT positioned near a back side of the penis P.

Next, the introduction port part 912b is widely expanded and opened and the penis P of a user is inserted into the introduction portion 912, and then the hand expanding and holding an attachment part 912a is released, whereby the attachment part 912a of the introduction portion 912 of the container body 910 is attached to the penis P in such a manner as to cover the same from above the detachment prevention member 930.

In this state, as illustrated in fig. 23, a force acting inward from the attachment part 912a is large at the protrusion region 932ba at both lower ends of the back covering portion 932 and a bending moment acts at the back covering portion 932, so that the center region 932bb having the smallest thickness of the back covering portion 932 is bent in a direction of protruding to a back side and accordingly the attachment insertion space MS is deformed, and thus a pressure on the urethra UT continues to be relieved.

Embodiment 6

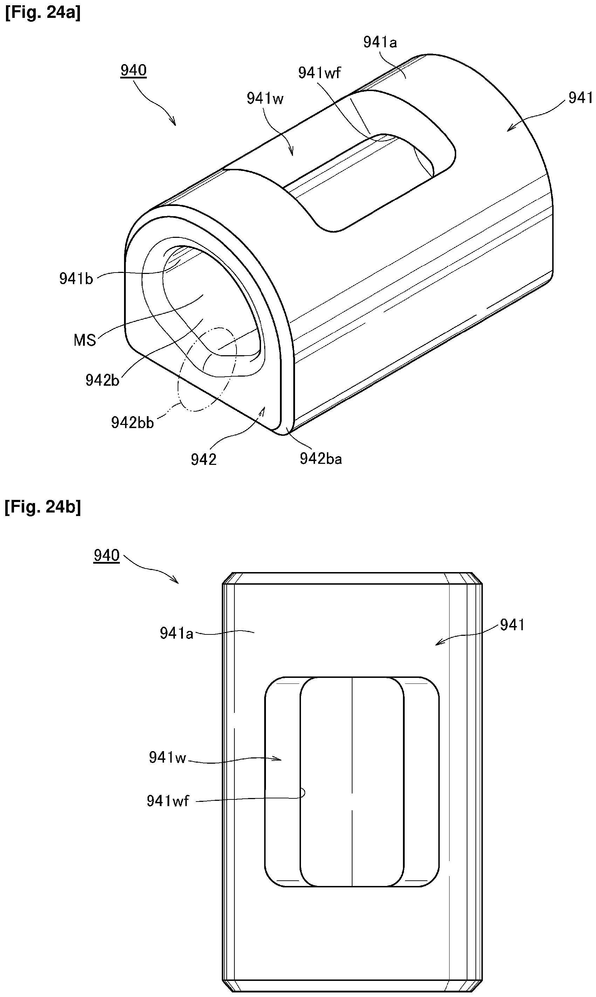

Note that if the detachment prevention member 930 according to the embodiment 5 as described above is attached while the foreskin F is loose near the base of the glans G of the penis P, the foreskin F returns toward the tip end of the glans G, which causes the glans G to relatively move from a large diameter region 914aa of the container body 910 to the introduction port part 912b, thereby creating a space near the large diameter region 914aa, so that the penis P may slip out of the container body 910, and therefore, next, a detachment prevention member 940 which can be used preferably even when the foreskin F of the penis P is loose will be described with reference to fig 24A to fig. 25.

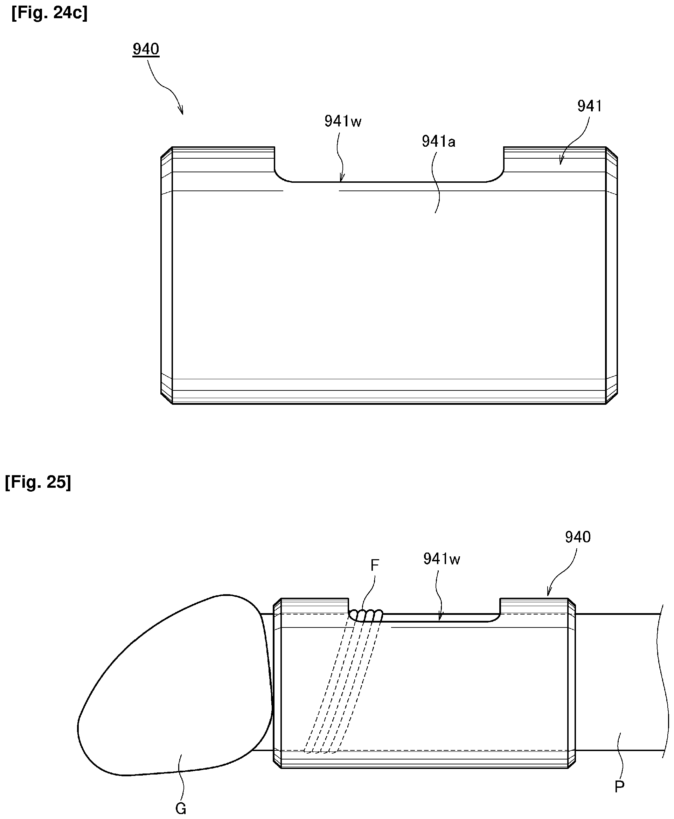

Then, fig. 24A is a schematic perspective view of a detachment prevention member according to a sixth embodiment of the present invention, fig. 24B is a schematic plan view of the detachment prevention member illustrated in fig. 24A, fig. 24C is a schematic side view of the detachment prevention member illustrated in fig. 24A, and fig. 25 is a schematic diagram illustrating a state in which the detachment prevention member illustrated in fig. 24A is attached to the penis.

The detachment prevention member 940 according to the present embodiment is used to prevent hindrance to urination and urine collection and further to prevent the container body 910 from detaching from the penis P by being attached to the penis P in advance when the foreskin F of the penis P is loose.

As illustrated in fig. 24A to fig. 24C, the detachment prevention member 940 includes a front covering portion 941 and a back covering portion 942 and is integrally formed from a flexible material.

The front covering portion 941 includes a semi-cylindrical front outer surface part 941a and a semi-cylindrical front inner surface part 941b and has a semi-cylindrical shape having a fixed thickness.

Further, the front covering portion 941 has a foreskin accommodation window part 941w in which the front covering portion 941 is configured to be open in a rounded rectangular shape at the center along the curvature of the semi-cylindrical front outer surface part 941a, the foreskin accommodation window part 941w being configured to communicate from the front inner surface part 941b to the front outer surface part 941a.

As illustrated in fig. 24A and fig. 24B, this foreskin accommodation window part 941w is open along the curvature of the front outer surface part 941a so as to be formed such that an opening area of the front outer surface part 941a is larger than an opening area of the front inner surface part 941b.

The back covering part 942 includes a flat back outer surface part 942a and a back inner surface part 942b having a substantially V-shaped cross-sectional shape and has such a shape that a thickness thereof decreases toward the center, and the back covering portion 942 has a protrusion region 942ba configured to protrude outward at both left and right ends as seen from an insertion direction and a center region 942bb having the smallest thickness.

To use the detachment prevention member 940, first, the penis P is inserted into the attachment insertion space MS of the detachment prevention member 940, and subsequently the foreskin F is moved toward the base of the penis P so as to be collected and accommodated in the foreskin accommodation window part 941w.

In this state, the back covering portion 942 includes the back inner surface part 942b having a substantially V-shaped cross-sectional shape and has such a shape that a thickness thereof decreases toward the center, so that at the center region 942bb, a pressure is relieved on the urethra UT positioned near a back side of the penis P.

Next, as illustrated in fig. 25, an attachment position of the detachment prevention member 940 and a state of the foreskin F of the penis P are adjusted such that the loose foreskin F securely remains within the foreskin accommodation window part 941w when the detachment prevention member 940 is attached to the penis P.

When the detachment prevention member 940 is thus attached to the penis P and then the container body 910 is further attached, the loose foreskin F is pressed by a window edge region 941wf and pinched within the foreskin accommodation window part 941w, which restricts and fixes the movement of the foreskin F.

Thus, by using the detachment prevention member 940 according to the present embodiment, it is possible to reliably prevent such inconvenience that the foreskin F returns toward the tip end of the glans G, which causes the glans G to relatively move from the large diameter region 914aa of the container body 910 to the introduction port part 912b, thereby creating a space near the large diameter region 914aa, so that the penis P slips out of the container body 910.

Further, it is possible to prevent such inconvenience that the foreskin F moves toward the tip end of the penis P together with the detachment prevention member 940 and covers the entire glans G to hinder urination and urine collection.

In addition, the foreskin accommodation window part 941w is provided in the front outer surface part 941a of the detachment prevention member 940, so that it is unnecessary to forcibly lower the foreskin F toward the base of the penis P to attach the detachment prevention member 940, which can reduce user discomfort.

## Reference Signs List

| | |
|---|---|
| 100 | biological fluid receptacle container |
| 110, 210, 810, 910 | container body |
| 111, 211, 311, 411 | receptacle portion |
| 111a | bulge part |
| 111aa, 211aa | thin-walled region (high elongation region) |
| 111b | receiving space bottom part |
| 112, 212, 912 | introduction portion |
| 112a, 212a, 912a | attachment part |
| 112b, 212b, 912b | introduction port part |
| 113, 213 | outlet portion |
| 113a, 213a | part to be fastened |
| 113b, 213b | outlet port part |
| 114, 214 | guide portion |
| 114a | support part |
| 114aa, 914aa | large diameter region |
| 114ab | small diameter region |
| 115, 215 | check valve portion |
| 115a | valve membrane part |
| 120, 520, 620, 720, 820, 920 | closure member |
| 121, 521, 621, 721, 821 | fastening ring portion |
| 521a, 721a | inner-ring protrusion portion |
| 122, 522, 622, 722, 822 | pressing portion |
| 623, 823 | closure member valve portion |
| 623a, 823a | closure member vale membrane part |
| 824 | outlet channel insertion portion |
| 825 | stopper protrusion portion |
| 826 | closure lid portion |
| 930, 940 | detachment prevention member |
| 931, 941 | front covering portion |
| 931a, 941a | front outer surface part |
| 931b, 941b | front inner surface part |
| 932, 942 | back covering portion |
| 932a, 942a | back outer surface part |
| 932b, 942b | back inner surface part |
| 932ba, 942ba | protrusion region |
| 932bb, 942bb | center region |

| 941w | foreskin accommodation window part |
|---|---|
| 941wf | window edge region |
| BF | biological fluid (urine) |
| AS | receiving space |
| IC | introduction channel |
| OC | outlet channel |
| GS | guiding space |
| VO | valve opening |
| AV | major axis of valve opening |
| IS | fastening insertion space |
| MS | attachment insertion space |
| TA | thickness dimension of receptacle portion |
| TI | thickness dimension of introduction portion |
| TO | thickness dimension of outlet portion |
| TG | thickness dimension of guide portion |
| TR | thickness dimension of detachment prevention member |
| LG | length dimension of guide portion |
| ID | inner diameter dimension of introduction channel |
| FO | closure member valve opening |
| OI | outlet portion insertion space |
| OS | outlet channel blocking space |
| VS | intervalve space |
| CC | closure member outlet channel |
| OO | closure member outlet port |
| B | human body |
| C | clothing |
| P | excretory organ (penis) |
| F | foreskin |
| G | glans |
| UO | external urethral orifice |
| AU | major axis of external urethral orifice |
| UT | urethra |
| θ | angle of deviation |

**Claims**

1. A container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, **characterized in that**

   the receptacle portion has such a flat shape as to have a bulge part in a lateral direction, and
   the check valve portion forms a valve opening having a slit shape such that substantially a lateral direction is a longitudinal direction.

2. A container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, **characterized in that**

a support part of the guide portion includes a large diameter region and a small diameter region formed at a position closer to the introduction portion than the large diameter region, and

a receiving space bottom part of the receptacle portion is arranged at a position corresponding to the small diameter region.

3. A container body configured by integrally forming from a material having a low hardness and a high elongation: a hollow receptacle portion configured to receive a biological fluid; a hollow introduction portion configured to communicate with one side of the receptacle portion and including an introduction port part for introducing the biological fluid; a hollow outlet portion configured to communicate with an other side of the receptacle portion and including an outlet port part for discharging the biological fluid; a hollow guide portion formed at a region at which the receptacle portion and the introduction portion communicate with each other toward an interior of the receptacle portion and configured to guide the biological fluid into the receptacle portion; and a check valve portion formed at the guide portion and configured to prevent backflow of the biological fluid, **characterized in that**

a minimum thickness dimension of the guide portion is configured to be smaller than a minimum thickness dimension of the introduction portion.

4. The container body according to any one of claim 1 to claim 3, **characterized in that** the receptacle portion includes a high elongation region having an elongation higher than an elongation of any region of the introduction portion and the outlet portion.

5. The container body according to any one of claim 1 to claim 3, **characterized in that** the receptacle portion, the guide portion, and the outlet portion are made of the same material, and

a minimum thickness dimension of the receptacle portion is configured to be smaller than any of a minimum thickness dimension of the guide portion and a minimum thickness dimension of the introduction portion.

6. The container body according to any one of claim 1 to claim 3, **characterized in that** the check valve portion includes a pair of valve membrane parts arranged facing each other and toward the receptacle portion at respective positions close to each other in a manner separate from each other.

7. The container body according to any one of claim 1 to claim 3, **characterized in that** the guide portion is configured to have a length dimension larger than half an inner diameter dimension of an introduction channel formed in the hollow introduction portion.

8. A detachment prevention member having a tubular shape, the detachment prevention member being configured to be used together with a biological fluid receptacle container including: a container body comprising at least a hollow receptacle portion configured to receive a biological fluid and a hollow introduction portion configured to communicate with one side of the receptacle portion and introduce the biological fluid; and a closure member, **characterized by** being configured by integrally forming from a flexible material: a front covering portion including a semi-cylindrical front outer surface part and a semi-cylindrical front inner surface part, the front covering portion having a semi-cylindrical shape having a fixed thickness; and a back covering portion including a flat back outer surface part and a back inner surface part having a substantially V-shaped cross-sectional shape, the back covering portion having such a shape that a thickness thereof decreases toward a center.

9. The detachment prevention member according to claim 8, **characterized in that** the front covering portion has a foreskin accommodation window part configured to communicate from the front inner surface part to the front outer surface part.

[Fig. 1]

[Fig. 2a]

[Fig. 2b]

[Fig. 3]

[Fig. 4]

[Fig. 5a]

[Fig. 5b]

[Fig. 6a]

[Fig. 6b]

[Fig. 7a]

[Fig. 7b]

[Fig. 8]

[Fig. 9]

310

315

314

[Fig. 10a]

410

415

414

[Fig. 10b]

410

415

414

BF

[Fig. 11]

[Fig. 12a]

[Fig. 12b]

[Fig. 12c]

[Fig. 13a]

[Fig. 13b]

[Fig. 14]

[Fig. 15]

[Fig. 16]

[Fig. 17]

[Fig. 18]

[Fig. 19]

[Fig. 20]

[Fig. 21]

[Fig. 22a]

[Fig. 22b]

[Fig. 23]

[Fig. 24a]

[Fig. 24b]

[Fig. 24c]

[Fig. 25]

<table>
<tr><td colspan="2">**INTERNATIONAL SEARCH REPORT**</td><td>International application No.<br>**PCT/JP2023/047253**</td></tr>
</table>

**A. CLASSIFICATION OF SUBJECT MATTER**

*A61F 5/453*(2006.01)i
FI: A61F5/453

According to International Patent Classification (IPC) or to both national classification and IPC

**B. FIELDS SEARCHED**

Minimum documentation searched (classification system followed by classification symbols)

A61F5/453

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2017-123999 A (FUJI LATEX CO.) 20 July 2017 (2017-07-20)<br>paragraphs [0015]-[0028], fig. 1-4 | 1-9 |
| A | JP 2013-099502 A (SEKIYAMA, Mihoko) 23 May 2013 (2013-05-23)<br>paragraphs [0021]-[0029], fig. 1, 2 | 1-9 |
| A | JP 11-513597 A (GOULTER, Barbara) 24 November 1999 (1999-11-24)<br>p. 15, fig. 1 | 1-9 |
| A | WO 2021/090621 A1 (INTRON SPACE INC.) 14 May 2021 (2021-05-14)<br>paragraphs [0029]-[0128], fig. 1-13 | 1-9 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 January 2024** | **27 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
**Information on patent family members**

| International application No. |
|---|
| **PCT/JP2023/047253** |

| Patent document cited in search report | | | Publication date (day/month/year) | Patent family member(s) | | | Publication date (day/month/year) |
|---|---|---|---|---|---|---|---|
| JP | 2017-123999 | A | 20 July 2017 | (Family: none) | | | |
| JP | 2013-099502 | A | 23 May 2013 | US | 2012/0165768 | A1 | |
| | | | | paragraphs [0044]-[0063], fig. 17, 18 | | | |
| JP | 11-513597 | A | 24 November 1999 | WO | 1997/014353 | A1 | |
| | | | | pp. 5, 6, fig. 1 | | | |
| | | | | US | 5797890 | A | |
| | | | | US | 5618277 | A | |
| | | | | KR | 1999-0066937 | A | |
| WO | 2021/090621 | A1 | 14 May 2021 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S581445 A
- JP 2016052393 A
- JP 2000157566 A
- JP 2004057747 A
- US 2005251100 A1
- US 5458114 A
- JP 3146490 U
- JP 2021074491 A